# EUROPEAN PATENT APPLICATION

(11) **EP 4 129 319 A1**
(43) Date of publication of application: **08.02.2023**
(21) Application number: 21189137.9
(22) Date of filing: 02.08.2021
(51) Int. Cl.: A61K 38/00, C07K 14/47

(54) **PORE-FORMING ANTIMICROBIAL PEPTIDES**

(71) Applicant: Masarykova univerzita, 60177 Brno (CZ)
(72) Inventor: Vacha, Robert, 61600 Brno (CZ); Deb, Rahul, Brno (CZ); Kabelka, Ivo, 66461 Holasice (CZ)
(74) Representative: Hartvichova, Katerina

(57) **Abstract**

The present invention provides a method for preparing pore-forming antimicrobial peptides, sequence patterns of pore-forming antimicrobial peptides which are obtained by the said method which are for antimicrobial use in vivo or in vitro.

## Description

### Field of Art

The present invention relates to structures of pore-forming antimicrobial peptides and to a method for producing antimicrobial peptides.

### Background Art

Increasing incidence of antibiotic resistance in bacteria along with a steady decline in the discovery of new antibiotics present a global health challenge. Currently, around 700,000 people die each year worldwide due to antibiotic-resistant infections, and, if no action is taken, the death rate is predicted to reach 10 million by 2050. Therefore, there is a need for the discovery of alternative class of antibiotics. Antimicrobial peptides (AMPs) have emerged as a promising candidate for such new antibiotics. Especially, the peptides which are able to form leaky transmembrane pores in the bacterial cytoplasmic membrane are of particular interest as they can directly kill the antibiotic-resistant bacteria.

Over the last few decades, a number of natural and synthetic membrane-permeabilizing AMPs with various pore-forming abilities have been discovered. However, fundamental rules for the guided development of pore-forming peptides are missing. The determination of such rules is challenging because a single amino acid substitution has been reported to be enough to cause a dramatic change in the pore structure or even completely change the peptide mode of action.

The most stable structure of transmembrane pores formed by AMPs is the barrel-stave pore. This pore structure is formed by peptides assembled in a helix bundle, just like the staves of a barrel. The helical peptides orient roughly perpendicular to the membrane plane and enable the formation of an aqueous channel. Membrane surrounding the pore is not significantly disrupted and lipids do not directly participate in formation of the pore structure. Two typical examples of barrel-stave pore-forming peptides are alamethicin, a 20-residues long AMP isolated from the fungus *Trichoderma viride* (Leitgeb et al. Chem. Biodivers. 2007, 4, 6, 1027-1051), and the synthetically derived LS3, a 21-residues long peptide composed of three heptameric repeats of leucine and serine (Lear et al. Science 1988, 240, 4856, 1177-1181).

The amphipathic character of peptides is essential for the barrel-stave pore formation. The hydrophobic regions on the peptide surface interact with the hydrophobic core of the bilayer and the hydrophilic regions form the interior of the pore (Brogden, K. A. Nat. Rev. Microbiol. 2005, 3, 3, 238-250). In addition, strong interhelical interactions between the neighboring transmembrane peptides are important for the stabilization of pore structure. For example, close packing of L side chains in a "knobs-into-holes" fashion has been reported to stabilize the LS3 ion channel (Lear et al. Science 1988, 240, 4856, 1177-1181). Further, aromatic π-π stacking interactions and GxxxG (SEQ ID NO. 290) motif were also reported to support the oligomerization of transmembrane helices. Additional pore stabilization can be achieved by a suitable placement of polar residues, which then interact via salt bridge interactions and water-mediated hydrogen bonds. Indeed, a recent study demonstrated the plausible interpeptide salt bridge interactions important in pore stability (Sepehri et al. Biophys. J. 2020, 118, 8, 1901-1913). However, it is still unclear how to combine the described effects and sequence patterns in the rational design of novel or more potent peptides.

### Disclosure of the Invention

The present invention is based on the discovery of design rules for α-helical antimicrobial peptides capable of forming stable barrel-stave pores. The inventors have developed such design rules based on the determined advantageous positions in the peptide sequence for pore-stabilizing intermolecular aromatic π-π stacking interactions and salt bridge interactions. The present invention provides a method of producing an antimicrobial peptide based on the novel peptide design rules, and antimicrobial peptide sequence patterns which are obtained by the said method.

A first aspect of the invention is an antimicrobial peptide comprising at least 22 amino acids, comprising a sequence selected from the group consisting of:
h KK hh KK hspsh pph a pph pa h (SEQ ID NO. 1)
h aa hh pp hspsh Kph K pKh pK h (SEQ ID NO. 2)
h KK hh KK hspsh pph a pah pp h (SEQ ID NO. 3)
h ap hh pa hspsh Kph K pKh pK h (SEQ ID NO. 4)
h KK hh KK hspsh aph h pph pa h (SEQ ID NO. 5)
h pa hh ap hspsh Kph K pKh pK h (SEQ ID NO. 6)
h KK hh KK hspsh aph h pah pp h (SEQ ID NO. 7)
h pp hh aa hspsh Kph K pKh pK h (SEQ ID NO. 8)
h KK ss KK hhphh pph s pph aa s (SEQ ID NO. 9)
h aa ss pp hhphh Kph s pKh KK s (SEQ ID NO. 10)
h KK ss KK hhphh pph s pah ap s (SEQ ID NO. 11)
h ap ss pa hhphh Kph s pKh KK s (SEQ ID NO. 12)
h KK ss KK hhphh aph s pph pa s (SEQ ID NO. 13)
h pa ss ap hhphh Kph s pKh KK s (SEQ ID NO. 14)
h KK ss KK hhphh aph s pah pp s (SEQ ID NO. 15)
h pp ss aa hhphh Kph s pKh KK s (SEQ ID NO. 16)
h KK hh KK hspsh aph a pah pa h (SEQ ID NO. 17)
h aa hh aa hspsh Kph K pKh pK h (SEQ ID NO. 18)
h KK hh KK hspsh aph h aah pa h (SEQ ID NO. 19)
h aa hh aa hspsh Kph h KKh pK h (SEQ ID NO. 20)
h KK hh KK hspsh aph h pah aa h (SEQ ID NO. 21)
h aa hh aa hspsh Kph h pKh KK h (SEQ ID NO. 22)
h KK hh KK hspsh pph a aah pa h (SEQ ID NO. 23)
h aa hh aa hspsh pph K KKh pK h (SEQ ID NO. 24)
h KK hh KK hspsh pph a pah aa h (SEQ ID NO. 25)
h aa hh aa hspsh pph K pKh KK h (SEQ ID NO. 26)
h KK hh KK hspsh pph h aah aa h (SEQ ID NO. 27)
h aa hh aa hspsh pph h KKh KK h (SEQ ID NO. 28)
h aa hh aa hspsh Kph K KKh KK h (SEQ ID NO. 29)
h KK ss KK hhphh aph s aah pa s (SEQ ID NO. 30)
h aa ss aa hhphh Kph s KKh pK s (SEQ ID NO. 31)
h KK ss KK hhphh aph s pah aa s (SEQ ID NO. 32)
h aa ss aa hhphh Kph s pKh KK s (SEQ ID NO. 33)
h KK ss KK hhphh pph s aah aa s (SEQ ID NO. 34)
h aa ss aa hhphh pph s KKh KK s (SEQ ID NO. 35)
h aa ss aa hhphh Kph s KKh KK s (SEQ ID NO. 36)
   wherein
   the sequences are written from N-terminus to C-terminus,
   wherein
   K is lysine;
   a is independently selected on each occurence from acidic amino acids selected from D (aspartic acid) and E (glutamic acid);
   h is independently selected on each occurence from hydrophobic amino acids selected from A (alanine), M (methionine), V (valine), L (leucine), and I (isoleucine);
   p is independently selected on each occurence from polar amino acids selected from T (threonine), Q (glutamine), S (serine) and N (asparagine);
   s is independently selected on each occurence from aromatic amino acids W (tryptophan) and F (phenylalanine);
   for use in treatment of diseases or infections caused by microbes.

The extension on N-terminus and/or C-terminus is preferably formed by at least one amino acid, said one or more amino acids are selected from K (lysine) and the amino acids of the groups described herein as a (acidic amino acids), b (basic amino acids, i.e. arginine or histidine), and p (polar amino acids).

Preferably, the sequence is extended on each terminus by at least four amino acids, wherein the amino acids are selected from K (lysine) and p (polar amino acids). Such extended sequences may then be further extended, for example by purification tags.

More preferably, the antimicrobial peptide of the present invention has the length of at least 30 amino acids and comprises a sequence selected from the group consisting of:
KKKKh KK hh KK hspsh pph a pph pa h KppK (SEQ ID NO. 37)
Kppph aa hh pp hspsh Kph K pKh pK h KKKK (SEQ ID NO. 38)
KKKKh KK hh KK hspsh pph a pah pp h KKKK (SEQ ID NO. 39)
KppKh ap hh pa hspsh Kph K pKh pK h KKKK (SEQ ID NO. 40)
KKKKh KK hh KK hspsh aph h pph pa h KppK (SEQ ID NO. 41)
KKpph pa hh ap hspsh Kph K pKh pK h KKKK (SEQ ID NO. 42)
KKKKh KK hh KK hspsh aph h pah pp h KKKK (SEQ ID NO. 43)
KKKKh pp hh aa hspsh Kph K pKh pK h KKKK (SEQ ID NO. 44)
KKKKh KK ss KK hhphh pph s pph aa s pppK (SEQ ID NO. 45)
Kppph aa ss pp hhphh Kph s pKh KK s KKKK (SEQ ID NO. 46)
KKKKh KK ss KK hhphh pph s pah ap s pKKK (SEQ ID NO. 47)
KppKh ap ss pa hhphh Kph s pKh KK s KKKK (SEQ ID NO. 48)
KKKKh KK ss KK hhphh aph s pph pa s KppK (SEQ ID NO. 49)
KKpph pa ss ap hhphh Kph s pKh KK s KKKK (SEQ ID NO. 50)
KKKKh KK ss KK hhphh aph s pah pp s KKKK (SEQ ID NO. 51)
KKKKh pp ss aa hhphh Kph s pKh KK s KKKK (SEQ ID NO. 52)
KKKKh KK hh KK hspsh aph a pah pa h KKKK (SEQ ID NO. 53)
KKKKh aa hh aa hspsh Kph K pKh pK h KKKK (SEQ ID NO. 54)
KKKKh KK hh KK hspsh aph h aah pa h KKKK (SEQ ID NO. 55)
KKKKh aa hh aa hspsh Kph h KKh pK h KKKK (SEQ ID NO. 56)
KKKKh KK hh KK hspsh aph h pah aa h KKKK (SEQ ID NO. 57)
KKKKh aa hh aa hspsh Kph h pKh KK h KKKK (SEQ ID NO. 58)
KKKKh KK hh KK hspsh pph a aah pa h KKKK (SEQ ID NO. 59)
KKKKh aa hh aa hspsh pph K KKh pK h KKKK (SEQ ID NO. 60)
KKKKh KK hh KK hspsh pph a pah aa h KKKK (SEQ ID NO. 61)
KKKKh aa hh aa hspsh pph K pKh KK h KKKK (SEQ ID NO. 62)
KKKKh KK hh KK hspsh pph h aah aa h KKKK (SEQ ID NO. 63)
KKKKh aa hh aa hspsh pph h KKh KK h KKKK (SEQ ID NO. 64)
KKKKh aa hh aa hspsh Kph K KKh KK h KKKK (SEQ ID NO. 65)
KKKKh KK ss KK hhphh aph s aah pa s KKKK (SEQ ID NO. 66)
KKKKh aa ss aa hhphh Kph s KKh pK s KKKK (SEQ ID NO. 67)
KKKKh KK ss KK hhphh aph s pah aa s KKKK (SEQ ID NO. 68)
KKKKh aa ss aa hhphh Kph s pKh KK s KKKK (SEQ ID NO. 69)
KKKKh KK ss KK hhphh pph s aah aa s KKKK (SEQ ID NO. 70)
KKKKh aa ss aa hhphh pph s KKh KK s KKKK (SEQ ID NO. 71)
KKKKh aa ss aa hhphh Kph s KKh KK s KKKK (SEQ ID NO. 72)
   wherein
   the sequences are written from N-terminus to C-terminus,
   wherein
   K is lysine;
   a is independently selected on each occurence from acidic amino acids selected from D (aspartic acid) and E (glutamic acid);
   h is independently selected on each occurence from hydrophobic amino acids selected from A (alanine), M (methionine), V (valine), L (leucine), and I (isoleucine);
   p is independently selected on each occurence from polar amino acids selected from T (threonine), Q (glutamine), S (serine), and N (asparagine);
   s is independently selected on each occurence from aromatic amino acids W (tryptophan) and F (phenylalanine).

A second aspect of the invention is the use of an antimicrobial peptide comprising a sequence selected from the group consisting of SEQ ID NO. 1-36, or preferably a sequence selected from the group consisting of SEQ ID NO. 37-72, for killing microbes *in vitro* (or *ex vivo*).

A third aspect of the invention is a method for producing an antimicrobial peptide, wherein the peptide comprises a sequence consisting of the following fragments: frag1A-frag2-frag3-frag4-frag5-frag6-frag7-frag8-frag9-frag10;
wherein
the sequences are written from N-terminus to C-terminus,
wherein:
   K is lysine;
   a is independently selected on each occurence from acidic amino acids selected from D (aspartic acid) and E (glutamic acid);
   h is independently selected on each occurence from hydrophobic amino acids selected from A (alanine), M (methionine),V (valine), L (leucine), and I (isoleucine);
   p is independently selected on each occurence from polar amino acids selected from T (threonine), Q (glutamine), S (serine), and N (asparagine);
   s is independently selected on each occurence from aromatic amino acids W (tryptophan) and F (phenylalanine).
   the said method comprising the steps:
      - determining the structure of fragment frag1A, wherein frag1A is h;
      - determining the structure of fragment frag2, wherein frag2 is selected from KK, aa, ap, pa, and pp;
      - determining the structure of fragments frag4 and frag6, wherein
         - when frag2 is KK, then frag4 is KK, and frag6 is pph or aph;
         - when frag2 is aa, then frag4 is pp or aa;
            - when frag4 is pp, then frag6 is Kph;
            - when frag4 is aa, then frag6 is pph or Kph;
         - when frag2 is ap, then frag4 is pa, and frag6 is Kph;
         - when frag 2 is pa, then frag4 is ap, and frag6 is Kph;
         - when frag2 is pp, then frag4 is aa, and frag6 is Kph;
      - determining the structure of fragment frag3, wherein frag3 is ss or hh;
      - determining the structure of fragments frag5 and frag10, wherein
         - when frag3 is ss, then frag5 is hhphh (SEQ ID NO. 73), and then frag10 is s;
         - when frag3 is hh, then frag5 is hspsh (SEQ ID NO. 74), and then frag10 is h;
      - determining the structure of fragment frag7, wherein
         - when frag3 is ss, then frag7 is s;
         - when frag3 is hh, then
            - when frag2 is KK, then frag7 is a or h;
            - when frag2 is aa and frag4 is pp, then frag7 is K;
            - when frag2 is aa and frag4 is aa, then frag7 is K or h;
            - when frag2 is ap, pa, or pp, then frag7 is K;
      - determining the structure of fragment frag8, wherein
         - when frag2 is KK and frag6 is pph and frag7 is s or a, then frag8 is pph, pah, or aah;
         - when frag2 is KK and frag6 is pph and frag7 is h, then frag8 is aah;
         - when frag2 is KK and frag6 is aph and frag7 is s or h, then frag8 is pph, pah, or aah;
         - when frag2 is KK and frag6 is aph and frag7 is a, then frag8 is pah;
         - when frag2 is aa and frag4 is pp, then frag8 is pKh;
         - when frag2 is aa and frag4 is aa and frag6 is pph and frag7 is s or h, then frag8 is KKh;
         - when frag2 is aa and frag4 is aa and frag6 is pph and frag7 is K, then frag8 is pKh or KKh;
         - when frag2 is aa and frag4 is aa and frag6 is Kph, then frag8 is pKh or KKh;
         - when frag2 is ap, pa, or pp, then frag8 is pKh;
      - determining the structure of fragment frag9, wherein
         - when frag2 is KK and frag6 is pph and frag7 is s and frag8 is pph, then frag9 is aa;
         - when frag2 is KK and frag6 is pph and frag7 is s and frag8 is pah, then frag9 is ap;
         - when frag2 is KK and frag6 is pph and frag7 is s and frag8 is aah, then frag9 is aa;
         - when frag2 is KK and frag6 is pph and frag7 is a and frag8 is pph, then frag9 is pa;
         - when frag2 is KK and frag6 is pph and frag7 is a and frag8 is pah, then frag9 is aa or pp;
         - when frag2 is KK and frag6 is pph and frag7 is a and frag8 is aah, then frag9 is pa;
         - when frag2 is KK and frag6 is pph and frag7 is h and frag8 is aah, then frag9 is aa;
         - when frag2 is KK and frag6 is aph and frag7 is s and frag8 is pph, then frag9 is pa;
         - when frag2 is KK and frag6 is aph and frag7 is s and frag8 is pah, then frag9 is aa or pp;
         - when frag2 is KK and frag6 is aph and frag7 is s and frag8 is aah, then frag9 is pa;
         - when frag2 is KK and frag6 is aph and frag7 is a and frag8 is pah, then frag9 is pa;
         - when frag2 is KK and frag6 is aph and frag7 is h and frag8 is pph, then frag9 is pa;
         - when frag2 is KK and frag6 is aph and frag7 is h and frag8 is pah, then frag9 is aa or pp;
         - when frag2 is KK and frag6 is aph and frag7 is h and frag8 is aah, then frag9 is pa;
         - when frag6 is Kph and frag7 is s and frag8 is pKh, then frag9 is KK;
         - when frag6 is Kph and frag7 is K and frag8 is pKh, then frag9 is pK;
         - when frag2 is aa and frag4 is aa and frag6 is pph and frag7 is s and frag8 is KKh, then frag9 is KK;
         - when frag2 is aa and frag4 is aa and frag6 is pph and frag7 is K and frag8 is pKh, then frag9 is KK;
         - when frag2 is aa and frag4 is aa and frag6 is pph and frag7 is K and frag8 is KKh, then frag9 is pK;
         - when frag2 is aa and frag4 is aa and frag6 is pph and frag7 is h and frag8 is KKh, then frag9 is KK;
         - when frag2 is aa and frag4 is aa and frag6 is Kph and frag7 is s and frag8 is KKh, then frag9 is pK or KK;
         - when frag2 is aa and frag4 is aa and frag6 is Kph and frag7 is K and frag8 is KKh, then frag9 is KK;
         - when frag2 is aa and frag4 is aa and frag6 is Kph and frag7 is h and frag8 is pKh, then frag9 is KK;
         - when frag2 is aa and frag4 is aa and frag6 is Kph and frag7 is h and frag8 is KKh, then frag9 is pK;
      - producing the peptide comprising a sequence of fragments frag1A-frag2-frag3-frag4-frag5-frag6-frag7-frag8-frag9-frag10, wherein the fragments are determined in the preceding steps.

In a preferred embodiment, the method is a method for producing an antimicrobial peptide, wherein the peptide comprises a sequence consisting of fragments: frag1-frag2-frag3-frag4-frag5-frag6-frag7-frag8-frag9-frag10-frag11; wherein
the sequences are written from N-terminal to C-terminal,
wherein:
   K is lysine;
   a is independently selected on each occurence from acidic amino acids selected from D (aspartic acid) or E (glutamic acid);
   h is independently selected on each occurence from hydrophobic amino acids selected from A (alanine) or M (methionine) or V (valine) or L (leucine) or I (isoleucine);
   p is independently selected on each occurence from upolar amino acids selected from T (threonine) or Q (glutamine) or S (serine) or N (asparagine);
   s is independently selected on each occurence from aromatic amino acids W (tryptophan) or F (phenylalanine).
   the said method comprising the steps:
      - determining the structure of fragments frag2, wherein frag2 is selected from KK, aa, ap, pa, and pp;
      - determining the structure of fragments frag4 and frag6, wherein
         - when frag2 is KK, then frag4 is KK, and frag6 is pph or aph;
         - when frag2 is aa, then frag4 is pp or aa;
            - when frag4 is pp, then frag6 is Kph;
            - when frag4 is aa, then frag6 is pph or Kph;
         - when frag2 is ap, then frag4 is pa, and frag6 is Kph;
         - when frag 2 is pa, then frag4 is ap, and frag6 is Kph;
         - when frag2 is pp, then frag4 is aa, and frag6 is Kph;
      - determining the structure of fragment frag3, wherein frag3 is ss or hh.
      - determining the structure of fragments frag5 and frag 10, wherein
         - when frag3 is ss, then frag5 is hhphh (SEQ ID NO. 73), and then frag10 is s;
         - when frag3 is hh, then frag5 is hspsh (SEQ ID NO. 74), and then frag10 is h;
      - determining the structure of fragment frag7, wherein
         - when frag3 is ss, then frag7 is s;
         - when frag3 is hh, then
            - when frag2 is KK, then frag7 is a or h;
            - when frag2 is aa and frag4 is pp, then frag7 is K;
            - when frag2 is aa and frag4 is aa, then frag7 is K or h;
            - when frag2 is ap, pa, or pp, then frag7 is K;
      - determining the structure of fragment frag8, wherein
         - when frag2 is KK and frag6 is pph and frag7 is s or a, then frag8 is pph, pah, or aah;
         - when frag2 is KK and frag6 is pph and frag7 is h, then frag8 is aah;
         - when frag2 is KK and frag6 is aph and frag7 is s or h, then frag8 is pph, pah, or aah;
         - when frag2 is KK and frag6 is aph and frag7 is a, then frag8 is pah;
         - when frag2 is aa and frag4 is pp, then frag8 is pKh;
         - when frag2 is aa and frag4 is aa and frag6 is pph and frag7 is s or h, then frag8 is KKh;
         - when frag2 is aa and frag4 is aa and frag6 is pph and frag7 is K, then frag8 is pKh or KKh;
         - when frag2 is aa and frag4 is aa and frag6 is Kph, then frag8 is pKh or KKh;
         - when frag2 is ap, pa, or pp, then frag8 is pKh;
      - determining the structure of fragment frag9, wherein
         - when frag2 is KK and frag6 is pph and frag7 is s and frag8 is pph, then frag9 is aa;
         - when frag2 is KK and frag6 is pph and frag7 is s and frag8 is pah, then frag9 is ap;
         - when frag2 is KK and frag6 is pph and frag7 is s and frag8 is aah, then frag9 is aa;
         - when frag2 is KK and frag6 is pph and frag7 is a and frag8 is pph, then frag9 is pa;
         - when frag2 is KK and frag6 is pph and frag7 is a and frag8 is pah, then frag9 is aa or pp;
         - when frag2 is KK and frag6 is pph and frag7 is a and frag8 is aah, then frag9 is pa;
         - when frag2 is KK and frag6 is pph and frag7 is h and frag8 is aah, then frag9 is aa;
         - when frag2 is KK and frag6 is aph and frag7 is s and frag8 is pph, then frag9 is pa;
         - when frag2 is KK and frag6 is aph and frag7 is s and frag8 is pah, then frag9 is aa or pp;
         - when frag2 is KK and frag6 is aph and frag7 is s and frag8 is aah, then frag9 is pa;
         - when frag2 is KK and frag6 is aph and frag7 is a and frag8 is pah, then frag9 is pa;
         - when frag2 is KK and frag6 is aph and frag7 is h and frag8 is pph, then frag9 is pa;
         - when frag2 is KK and frag6 is aph and frag7 is h and frag8 is pah, then frag9 is aa or pp;
         - when frag2 is KK and frag6 is aph and frag7 is h and frag8 is aah, then frag9 is pa;
         - when frag6 is Kph and frag7 is s and frag8 is pKh, then frag9 is KK;
         - when frag6 is Kph and frag7 is K and frag8 is pKh, then frag9 is pK;
         - when frag2 is aa and frag4 is aa and frag6 is pph and frag7 is s and frag8 is KKh, then frag9 is KK;
         - when frag2 is aa and frag4 is aa and frag6 is pph and frag7 is K and frag8 is pKh, then frag9 is KK;
         - when frag2 is aa and frag4 is aa and frag6 is pph and frag7 is K and frag8 is KKh, then frag9 is pK;
         - when frag2 is aa and frag4 is aa and frag6 is pph and frag7 is h and frag8 is KKh, then frag9 is KK;
         - when frag2 is aa and frag4 is aa and frag6 is Kph and frag7 is s and frag8 is KKh, then frag9 is pK or KK;
         - when frag2 is aa and frag4 is aa and frag6 is Kph and frag7 is K and frag8 is KKh, then frag9 is KK;
         - when frag2 is aa and frag4 is aa and frag6 is Kph and frag7 is h and frag8 is pKh, then frag9 is KK;
         - when frag2 is aa and frag4 is aa and frag6 is Kph and frag7 is h and frag8 is KKh, then frag9 is pK;
      - determining the structure of fragment frag 1, wherein
         - when frag2 is KK or pp, then frag1 is KKKKh (SEQ ID NO. 75);
         - when frag2 is aa and frag4 is aa, then frag1 is KKKKh (SEQ ID NO. 75);
         - when frag2 is aa and frag4 is pp, then frag1 is Kppph (SEQ ID NO. 76);
         - when frag2 is ap, then frag1 is KppKh (SEQ ID NO. 77);
         - when frag2 is pa, then frag1 is KKpph (SEQ ID NO. 78);
      - determining the structure of fragment frag 11, wherein
         - when frag8 is pph and frag9 is aa, then frag11 is pppK (SEQ ID NO. 79);
         - when frag8 is pph and frag9 is pa, then frag11 is KppK (SEQ ID NO. 80);
         - when frag8 is pah and frag9 is ap, then frag11 is pKKK (SEQ ID NO. 81);
         - when frag8 is pah or aah and frag9 is aa or pa, then frag11 is KKKK; (SEQ ID NO. 82);
         - when frag9 is pp or pK or KK, then frag11 is KKKK (SEQ ID NO. 82);
      - producing the peptide comprising a sequence of fragments frag1-frag2-frag3-frag4-frag5-frag6-frag7-frag8-frag9-frag10-frag11, wherein the fragments are determined in the preceding steps.

### Brief Description of Drawings

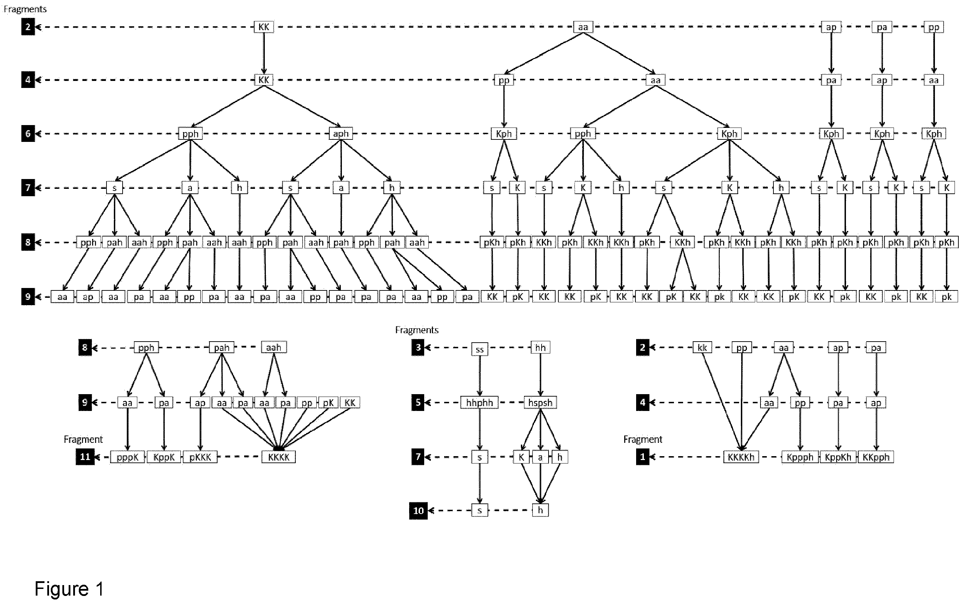

Figure 1 is a schematic representation of the algorithm for creating the sequence of peptides able to form barrel-stave pores with antimicrobial properties according to the method described herein.

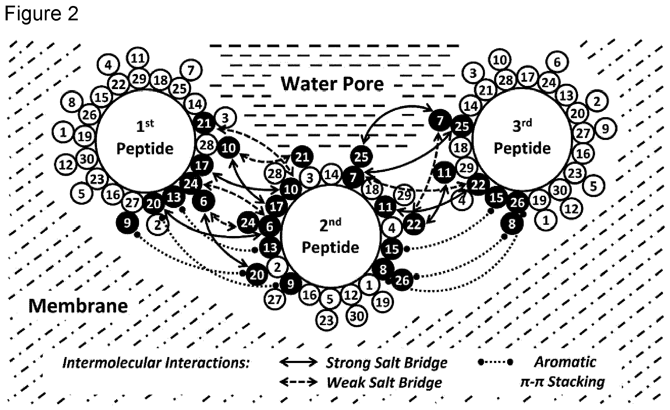

Figure 2 is a schematic illustration of the designed intermolecular salt bridge and aromatic π-π stacking interactions between the transmembrane peptides in barrel-stave pore. Three antiparallel neighboring peptides with two representative peptide-peptide interfaces inside the octameric pore are shown in top view helical wheel representations.

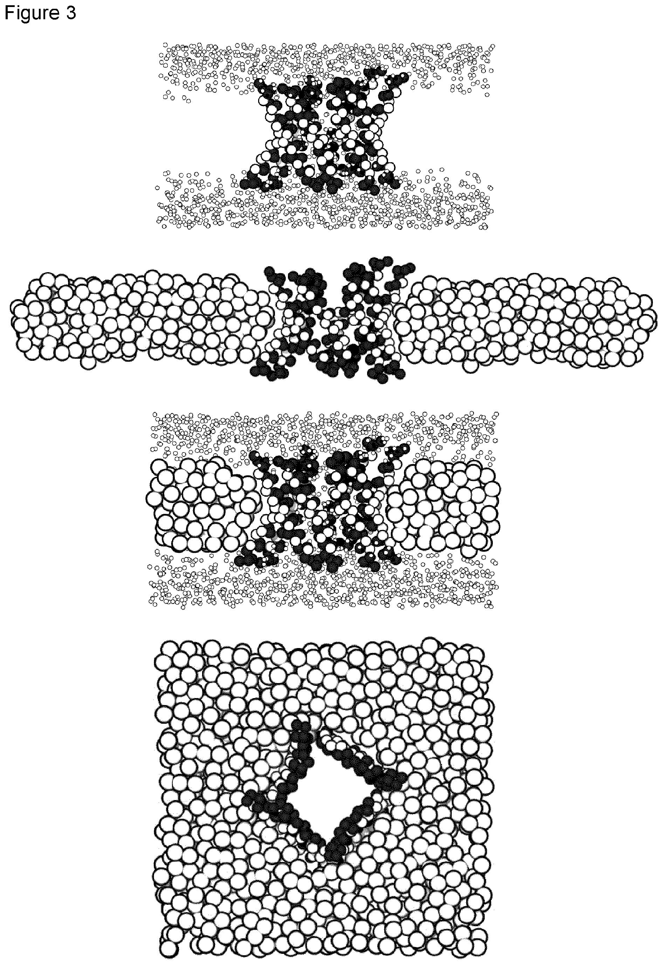

Figure 3 is a snapshot taken at the end of 51 µs simulation depicting the water channel inside a stable octameric barrel-stave pore formed by RDFA2 peptides (see Table 23 for the sequence) in a symmetric membrane composed of 1-palmitoyl-2-oleoyl-sn-glycero-3-phosphocholine (POPC) lipids. Peptide backbones are colored white and black for hydrophobic and hydrophilic residues, respectively; water molecules are shown in small white beads; and the membrane is represented only by the lipid tails (for clarity) shown in large white beads. The figure shows (in the indicated order): side view of the water channel inside the pore; side view of the same pore within membrane; side view of the same membrane pore with the displayed water channel; top view of the same pore structure.

### Detailed description of the Invention

The length of the antimicrobial peptide is preferably up to 50 amino acids, more preferably up to 40 amino acids, even more preferably up to 30 amino acids. The sequences SEQ ID NO. 1-36, or the sequences 37-72, or sequences obtained by the method of the invention may be extended on each terminus by at most 14 amino acids, preferably by at most 9 amino acids, even more preferably by at most 4 amino acids. The amino acids forming the extensions are preferably selected from K (lysine) and the amino acids of the groups described herein as a (acidic amino acids), b (basic amino acids, i.e. arginine or histidine), p (polar amino acids), proline and glycine more preferably selected from K (lysine) and polar amino acids (group p).

In some embodiments, the extension sequences may include at least one purification tag, such as ALFA-tag, AviTag, C-tag, polyglutamate tag, polyarginine tag, E-tag, FLAG-tag, HA-tag, His-tag, Myc-tag, NE-tag, RholD4-tag, S-tag, Softag1, Softag3, Spot-tag, Strep-tag, T7-tag, TCtag, Ty tag, V5 tag, VSV-tag, Xpress tag, Isopeptag, SpyTag, SnoopTag, DogTag, SdyTag.

In some embodiments, the extension sequences may include 1-5 lysines.
In some embodiments, the C-terminus of the compound may be amidated.

In the method, the steps of selecting and determining fragments, leading to the creation of the sequence, are preferably computer-implemented.

The following sequences are preferred for potent antimicrobial activity:
KKKKLKKILKKLFQFINQLDNQLQDIKQNK (SEQ ID NO. 83) which is a representative sequence of the general pattern SEQ ID NO. 37,
KKKKLKKILKKLWQWINQLDNQLQDIKQNK (SEQ ID NO. 84) a representative sequence of the general pattern SEQ ID NO. 37,
KKKKLKKILKKLFQFINQLENQLQEIKQNK (SEQ ID NO. 85) which is a representative sequence of the general pattern SEQ ID NO. 37,
KKKKLKKILKKLFQFINQLDNQLQDFKQNK (SEQ ID NO. 86) which is a representative sequence of the general pattern SEQ ID NO. 37,
KKKKLKKILKKLFQFINQLDNQLQDIKKKK (SEQ ID NO. 87) which is a representative sequence of the general pattern SEQ ID NO. 37,
KKKKKLKKILKKLFQFINQLDNQLQDIKKKKK (SEQ ID NO. 88) which is a representative sequence of the general pattern SEQ ID NO. 37 with extension by K residues at both termini,
KKKKLKKILKKLFQFIDQLDNDLQDIKKKK (SEQ ID NO. 89) which is a representative sequence of the general pattern SEQ ID NO. 53.

The invention development started with coarse-grained molecular dynamics simulations using Martini force field. At the beginning, the inventors investigated the peptide ability to stabilize a preformed octameric membrane pore created in the center of a pre-equilibrated 1-palmitoyl-2-oleoyl-sn-glycero-3-phosphocholine (POPC) bilayer (see Materials and Methods for details). The focus was on α-helical peptides with a secondary amphipathic character and the antiparallel arrangements of transmembrane peptides in pore, which was shown to be favored over the parallel arrangement.
Firstly, the pore-stabilizing effects of hydrophobic residues were investigated in 17 model peptides, P1-P17 (Table 1), which had varying amounts of hydrophobicity including the aromatic moieties. The half-hydrophobic model peptides P1 and P2, with the sequence patterns LLQQ (SEQ ID NO. 90) and LIQN (SEQ ID NO. 91), respectively, were not able to stabilize the pore for more than 5 µs. Even the peptides with lower (23%), P3, or higher (77%), P4, amount of hydrophobicity (i.e. size of the hydrophobic patch on the folded peptide) did not stabilize membrane pores. Therefore, the aromatic tryptophan (W) and phenylalanine (F) residues were introduced in P5 and P6, respectively, by replacing L/I in P2. P5 was able to stabilize the pore for longer time compared to P6, because of stronger peptide-peptide interactions. Further expansion of the aromatic patch in P7, i.e., mutating L/I-to-W in P4, resulted in the stabilization of a deformed barrel-stave pore till the end of simulation (51 µs). In this pore, all the eight peptides remained in the transmembrane state surrounding a narrow water channel, which suggested the possible pore stabilization via aromatic peptide-peptide interactions.

**Table 1. Investigated peptides with systematically varied hydrophobic amino acids in presence or in absence of the hydrophilic charged termini and their abilities to stabilize preformed pores in membrane composed of 1-palmitoyl-2-oleoyl-sn-glycero-3-phosphocholine (POPC) lipids. TMP stands for the number of transmembrane peptides at the end of simulation.**

| **Peptides** | **Primary structures** | **Mutations** | **Sim. time** | **Pore stability** | **TMP** |
|---|---|---|---|---|---|
| P1 | LLQQLQQLLQQLLQLLQQLLQQLQQLLQQL (SEQ ID NO. 92) | LLQQ pattern (SEQ ID NO. 90) | 2 µs | Closed | 3 |
| P2 | LIQNLQNILNQLIQLINQLINQLQNILQNL (SEQ ID NO. 93) | LIQN pattern (SEQ ID NO. 91) | 5 µs | Closed | 3 |
| P3 | QNQNLQNQLNQLNQNINQNQNQLQNQLQNL (SEQ ID NO. 94) | P2 more hydrophilic | 0.3 µs | Closed | 3 |
| P4 | LIQILLNILNILIQLILILINILLNILQIL (SEQ ID NO. 95) | P2 more hydrophobic | 5 µs | Closed | 3 |
| P5 | **WW**QN**W**QN**WW**NQ**WW**Q**WW**NQ**WW**NQ**W**QN**WW**QN**W** (SEQ ID NO. 96) | P2 L/I → W | 48 µs | Closed | 3 |
| P6 | **FF**QN**F**QN**FF**NQ**FF**Q**FF**NQ**FF**NQ**F**QN**FF**QN**F** (SEQ ID NO. 97) | P2 L/I → F | 8 µs | Closed | 3 |
| P7 | **WW**Q**WWW**N**WW**N**WWW**Q**WWWWWW**N**WWW**N**WW**Q**WW** (SEQ ID NO. 98) | P4 L/I → W | 51 µs | Deformed | 8 |
| P8 | **WW**QNLQNILNQLIQLINQLINQLQNILQ**WW** (SEQ ID NO. 99) | P2 L/I/N (1, 2, 29, 30)→W | 51 µs | Deformed | 4 |
| P9 | LIQNLQNILNQLIQ**WW**NQLINQLQNILQNL (SEQ ID NO. 100) | P2 L/I (15, 16) → W | 16 µs | Closed | 3 |
| P10 | LIQNLQNILNQ**WW**Q**WW**NQ**WW**NQLQNILQNL (SEQ ID NO. 101) | P2 L/I (12, 13, 15, 16, 19, 20) → W | 51 µs | Deformed | 5 |
| P11 | LIQNLQNILNQ**W**IQL**W**NQL**W**NQLQNILQNL (SEQ ID NO. 102) | P2 L/I (12, 16, 20) → W | 45 µs | Closed | 3 |
| P12 | LIQNLQNILNQL**W**QL**W**NQ**W**INQLQNILQNL (SEQ ID NO. 103) | P2 L/I (13, 16, 19) → W | 24 µs | Closed | 3 |
| P13 | LIQNLQNILNQ**FF**Q**FF**NQ**FF**NQLQNILQNL (SEQ ID NO. 104) | P10 W → F | 24 µs | Closed | 3 |
| P14 | LIQNLQNILNQ**F**IQL**F**NQL**F**NQLQNILQNL (SEQ ID NO. 105) | P11 W → F | 21 µs | Closed | 3 |
| P15 | LIQNLQNILNQL**F**QL**F**NQ**F**INQLQNILQNL (SEQ ID NO. 106) | P12 W → F | 51 µs | Stable | 6 |
| P16 | LIQNLQNILNQL**W**QLINQ**W**INQLQNILQNL (SEQ ID NO. 107) | P2 L/I (13, 19) → W | 51 µs | Stable | 6 |
| P17 | LIQNLQNILNQL**F**QLINQ**F**INQLQNILQNL (SEQ ID NO. 108) | P2 L/I (13, 19) → F | 33 µs | Closed | 3 |
| P18 | LIQNLQNILNQL**W**Q**W**INQLINQLQNILQNL (SEQ ID NO. 109) | P2 L/I (13, 15) → W | 2 µs | Closed | 3 |
| P19 | LIQNLQNILNQL**F**Q**F**INQLINQLQNILQNL (SEQ ID NO. 110) | P2 L/I (13, 15) → F | 10 µs | Closed | 3 |
| P20 | **NQRR**LQNILNQL**W**Q**W**INQLINQLQNI**RRQN** (SEQ ID NO. 111) | P18 Q/N/L (3,4,27,28) → R | 51 µs | Deformed | 4 |
| P21 | **NQRR**LQNILNQL**F**Q**F**INQLINQLQNI**RRQN** (SEQ ID NO. 112) | P19 Q/N/L (3,4,27,28) → R | 51 µs | Stable | 7 |
| P22 | LIQNLQN**WW**NQLIQLINQL**W**NQLQN**W**LQNL (SEQ ID NO. 113) | P2 L/I (8, 9, 20, 26) → W | 41 µs | Closed | 3 |
| P23 | LIQNLQN**FF**NQLIQLINQL**F**NQLQN**F**LQNL (SEQ ID NO. 114) | P2 L/I (8, 9, 20, 26) → F | 6 µs | Closed | 3 |
| P24 | **NQRR**LQN**WW**NQLIQLINQL**W**NQLQN**WRRQN** (SEQ ID NO. 115) | P22 Q/N/L (3,4,27,28) → R | 51 µs | Stable | 8 |
| P25 | **NQRR**LQN**FF**NQLIQLINQL**F**NQLQN**FRRQN** (SEQ ID NO. 116) | P23 Q/N/L (3,4,27,28) → R | 51 µs | Stable | 8 |
| P26 | **RRQN**LQN**WW**NQLIQLINQL**W**NQLQN**WNQRR** (SEQ ID NO. 117) | P22 L/I/N (1,2,29,30) → R | 51 µs | Stable | 8 |
| P27 | **RRQN**LQN**FF**NQLIQLINQL**F**NQLQN**FNQRR** (SEQ ID NO. 118) | P23 L/I/N (1,2,29,30) → R | 51 µs | Stable | 8 |

Increasing the overall hydrophobicity of the peptides beyond an optimum hydrophobicity has been shown to decrease the antimicrobial specificity. Therefore, rather than increasing the number of hydrophobic residues further, the inventors focused on the effect of distribution of aromatic residues in the peptides P8-P17. P8 with Ws at both the termini resulted in a deformed pore with an intermittent water channel till the end of simulation (51 µs). Detailed analysis revealed that P8 stabilized peptide-peptide interactions at the peptide termini, but the pore structure was not in shape of a barrel-stave pore as the peptide termini moved to the middle of the membrane. P9 with Ws in the middle of the sequence did not stabilize the pore either. The reason was that the W residues were too close to each other and remained intramolecularly stacked within the peptide. Therefore, more positions within the sequence were investigated for the formation of inter- and intramolecular aromatic π-π stacking (ST) interactions in the peptides P10-P17.
It was found that aromatic residues need to be in specific positions to stabilize the pore by intermolecular ST interactions. The presence of aromatic residues at other positions was found to be unfavorable due to intramolecular ST (compare P10-P14 with P15 and P16 in Table 1). Aromatic residues at positions 13 and 19 were identified to be ideally located for single intermolecular ST interaction on each peptide-peptide interface (Table 2). However, an additional aromatic residue at position 16 hindered 13-13 and 19-19 intermolecular ST by introducing intramolecular ST (see P12 in Table 1). The specific role of the positions 13 and 19 was found to be a consequence of the transmembrane peptide tilt in the pore. The tilt was on average 50 degrees from the membrane normal for the peptides P16 and P17.

**Table 2. Time-averaged numbers of intermolecular π-π stacking (ST) interactions in pores stabilized by the peptides with systematically varied posititions and types of aromatic residues (Table 1) investigated in simulations with preformed pore using POPC membrane.**

| **Peptides** | **Single ST per peptide-peptide interface** | | | **Double ST per peptide-peptide interface** | |
|---|---|---|---|---|---|
| | **13-13** | **15-15** | **19-19** | **9-20** | **8-26** |
| P16 | 2.5 | - | 2.6 | - | - |
| P17 | 2.0 | - | 2.4 | - | - |
| P20 | 1.6 | 0.7 | - | - | - |
| P21 | 2.7 | 1.3 | - | - | - |
| P22 | - | - | - | 1.7 | 6.6 |
| P24 | - | - | - | 4.8 | 7.7 |
| P25 | - | - | - | 4.6 | 7.3 |
| P26 | - | - | - | 6.3 | 6.5 |
| P27 | - | - | - | 5.3 | 6.2 |

Positively charged residues at each peptide terminus could provide additional stability to the barrel-stave pore structure, especially in the negatively charged bacterial membranes. Therefore, the inventors explored the pore-stabilizing effects of intermolecular ST interactions in the presence of cationic termini in the peptides P18-P21 (Table 1). Positively charged residues were introduced at the termini of P20 and P21 by mutating the second last two residues of P18 and P19 (i.e., 3-4 at N-terminus and 27-28 at C-terminus) to arginines (Rs), respectively. These substitutions reduced the length of the consecutive hydrophobic patch on the surface of the folded peptide running from 30 to 22 residues, which changed the transmembrane peptide tilt in the pore from 50 to 30 degrees. Subsequently, the modified tilt resulted in a shift of the preferred positions in the sequence for aromatic residues from 13 and 19 to 13 and 15, respectively (Table 2). As a result, both P20 and P21 managed to hold the pore structure till 51 µs, while P18 and P19, lacking the hydrophilic termini, did not stabilize the pore beyond 10 µs, despite having aromatic residues at positions 13 and 15.
Next, the inventors explored the effects of two intermolecular ST interactions on each interface in the peptides P22-P27 (Table 1). Peptides P24-P27 had different distributions of positively charged residues at the termini, whicle P22-P23 had hydrophobic residues at the termini. To prevent intramolecular ST interactions, the aromatic residues were moved from the peptide center to the terminus, while avoiding the very ends (tested previously in P8). Dual intermolecular ST interaction per interface was tested between the aromatic residues at positions 9 and 20 on the first interface and positions 8 and 26 on the second interface. Such dual ST interactions were found to be excellent for pore stabilization (till the end of simulations, i.e., 51 µs) for all the four peptides P24-P27 (Table 2), which had an average tilt of 30 degrees from the membrane normal. The same distribution of aromatic residues did not stabilize pores made of P22 and P23, which lack the hydrophilic termini and had an average tilt of 50 degrees. Moreover, there was no significant difference in the pore structures and stabilities of ST interactions for the different distribution of basic residues within the hydrophilic peptide termini (i.e., whether NQRR (SEQ ID NO. 119) or RRQN (SEQ ID NO. 120)).
The above results demonstrated the importance of the positions 13 and 15 in the sequence for single intermolecular ST interactions and the positions 8, 9, 20, and 26 for dual intermolecular ST interactions on each peptide-peptide interface, resulting in the enhanced stabilization of barrel-stave pores formed by the peptides with a continuous hydrophobic patch spanning across 22 amino acids.
Close packing of the polar side chains has been demonstrated to play an important role in the barrel-stave pore stabilization. Therefore, the inventors subsequently increased pore stability by introducing salt bridge (SB) interactions on the polar side of amphipathic peptides. More than 60 peptides were designed and tested with different combinations of intermolecular SB interactions (Table 3 and 4) based on the template peptides P20-P21 (with single ST per interface) and P24-P25 (with dual ST per interface).
Peptide nomenclature **RD(W/F)(A/B/C/D)(Sr. no.)(Roman no.)** is primarily based on the one letter code of the interacting amino acids, i.e., arginine, R, and aspartic acid, D, forming SB interactions and tryptophan, W, or phenylalanine, F, forming ST interactions. The fourth letter A/B/C/D denotes one of the four groups in which the peptides are split by the total number of ST and SB interactions per interface. For easier identification, the peptides are labeled with the serial numbers (1, 2, 3 ...) in Table 3. Table 4 contains peptides from Table 3 with further mutations, which are labeled additionally with the roman numbers (i, ii, iii ...). All the sequences can be found in Table 23. Pairs of oppositely charged residues at sequence positions 6-17, 6-20, 6-21, 6-24, 10-17, 10-20, 10-21, 10-24, and 14-17 were tested for SB formation on the first interface, and the pairs at the positions 7-18, 7-22, 7-25, 11-18, 11-22, and 11-25 were tested on the second interface. Because the position 20 is located at the peptide-peptide interface, this particular position was investigated also for SB interactions, despite of its original hydrophobic character and suitability for 9-20 double ST interactions.

**Table 3. Four groups of peptides designed with the combination of intermolecular stacking (ST) and salt bridge (SB) interactions on each peptide-peptide interface. All peptides have positively charged hydrophilic termini. Types of simulation studies are indicated as follows - C: preformed pore in POPC membrane, N: preformed pore in 1-palmitoyl-2-oleoyl-sn-glycero-3 -phosphoethanolamine : 1-palmitoyl-2-oleoyl-sn-glycero-3 -phospho-(1'-rac-glycerol) (POPE:POPG) (3:1 mol/mol) membrane, S: preformed pore in POPC membrane with the scaled Martini force filed, P: spontaneous pore formation and U: umbrella sampling of peptide adsorption on the membrane plane. Average number of water MARTINI beads inside the pores and peptide-peptide interaction energies were calculated in type C simulation over the last 3 µs (i.e., 48-51 µs) and for the entire 51 µs simulation, respectively.**

| **Peptides** | **Pairs investigated for SB formation** | | **Simulation types** | **Water inside RDW/F pore (Average)** | **P-P int. energy RDW/F (Average in kJ/mol)** |
|---|---|---|---|---|---|
| | **First peptide-peptide interface** | **Second peptide-peptide interface** | | | |
| **Group A:** One 13-13 ST (interface 1) or one 15-15 ST (interface 2) and two SB (total 3) interactions per interface | | | | | |
| RD(W/F)A1 | 6-17 | 7-18 | C | 21/23 | -9100 / -8500 |
| RD(W/F)A2 | 6-20 | 7-25 | CNSPU | 51/47 | -9200 / -9200 |
| RD(W/F)A3 | 6-21 | 7-22 | CS | 42/35 | -9400 / -9100 |
| RD(W/F)A4 | 6-24 | 7-25 | CS | 37/35 | -9500 / -9100 |
| RD(W/F)A5 | 10-17 | 11-22 | CNS | 41/36 | -9300 / -8900 |
| RD(W/F)A6 | 10-20 | 11-18 | C | 46/42 | -9300 / -9200 |
| RD(W/F)A7 | 10-21 | 11-22 | CNS | 29/25 | -9300 / -9500 |
| RD(W/F)A8 | 10-24 | 11-25 | C | 37/35 | -9400 / -9000 |
| RDWA9 | 14-17 | 11-18 | C | 31 | -9400 |

| **Group B:** Two 9-20 ST (interface 1) or two 8-26 ST (interface 2) and two SB (total 4) interactions per interface | | | | | |
|---|---|---|---|---|---|
| RD(W/F)B10 | 6-17 | 7-22 | C | 38/30 | -10600 / -10000 |
| RD(W/F)B11 | 6-21 | 7-22 | C | 31/25 | -10900 /-10100 |
| RD(W/F)B12 | 6-24 | 7-25 | C | 32/29 | -11100 /-10500 |
| RD(W/F)B13 | 10-17 | 11-22 | CN | 22/19 | -11000 / -10600 |
| RD(W/F)B14 | 10-21 | 11-22 | C | 21/18 | -10800 / -10400 |
| RD(W/F)B15 | 10-24 | 11-22 | C | 22/20 | -11100 / -10700 |

| **Group C:** One 13-13 ST (interface 1) or one 15-15 ST (interface 2) and four SB (total 5) interactions per interface | | | | | |
|---|---|---|---|---|---|
| RD(W/F)C16 | 6-17 & 10-20 6-20 & 10-17 | 7-22 & 11-25 \| 7-25 & 11-22 | CNSPU | 45/37 | -10200 / -10000 |
| RD(W/F)C17 | 6-17 & 10-21 6-21 & 10-17 | | C | 31/23 | -10200 / -10300 |
| RD(W/F)C18 | 6-17 & 10-24 6-24 & 10-17 | | C | 34/26 | -10400 /-10100 |
| RD(W/F)C19 | 6-20 & 10-21 6-21 & 10-20 | | CNSP | 28/33 | -10700 / -10000 |
| RD(W/F)C20 | 6-20 & 10-24 6-24 & 10-20 | | C | 45/35 | -10200 /-10100 |
| RD(W/F)C21 | 6-21 & 10-24 6-24 & 10-21 | | CNSU | 20/23 | -11000 / -10800 |

| **Group D:** Two 9-20 ST (interface 1) or two 8-26 ST (interface 2) and four SB (total 6) interactions per interface | | | | | |
|---|---|---|---|---|---|
| RD(W/F)D22 | 6-17 & 10-21 6-21 & 10-17 | 7-22 & 11-25 \| 7-25 & 11-22 | C | 23/19 | -11900 / -11500 |
| RD(W/F)D23 | 6-17 & 10-24 6-24 & 10-17 | | CN | 25/25 | -11600 / -11200 |
| RD(W/F)D24 | 6-21 & 10-24 6-24 & 10-21 | | CS | 22/16 | -11800 / -11500 |

**Table 4. Peptides designed (based on the template peptides from Table 3) to investigate the additional combinations of intermolecular stacking (ST) and salt bridge (SB) interactions in the pore and the effects of mutations in the hydrophilic termini. All peptides were studied in the simulations with a preformed pore in POPC membrane. The mutated SB are highlighted in bold. Average number of water molecules inside the pores and peptide-peptide interaction energies were calculated over the last 3 µs (i.e., 48-51 µs) and for the entire 51 µs simulation, respectively.**

| **Peptides** | **Pairs investigated for SB formation** | | **Mutations in the peptide termini** | **Water inside RDW/F pore (Average)** | **P-P int. energy RDW/F (Average in kJ/mol)** |
|---|---|---|---|---|---|
| | **First peptide-peptide interface** | **Second peptide-peptide interface** | | | |
| **Group A** | | | | | |
| RDW/FA1**i** | 6-17 | 7-**22** | - | 42/18 | -9300 / -9000 |
| RDW/FA1**ii** | 6-17 | **11-22** | - | 27/23 | -9100 / -8600 |
| RDFA2**i** | 6-20 | 7-25 | R28→Q, N30→R | 47 | -9000 |
| RDFA2**ii** | 6-20 | 7-25 | R28→N | 46 | -8800 |
| RDFA2iii | 6-20 | 7-25 | N/Q(1, 2, 29, 30)→R, | 35 | -8800 |
| | | | R(3, 4, 27, 28)→N/Q | | |
| RDW/FA2**iv** | 6-20 | 7-**22** | - | 51/44 | -9400 / -9000 |
| RDW/FA2**v** | 6-20 | **11-22** | - | 46/46 | -9500 / -8900 |
| RDFA4**i** | 6-24 | 7-25 | N(1,30)→R, R(27,28)→N/Q | 29 | -9500 |
| RDFA4**ii** | 6-24 | 7-25 | N(1,30)→R, R(3,27,28)→N/Q | 33 | -9100 |
| RDFA4i**ii** | 6-24 | 7-25 | N/Q(1, 2, 29, 30)→R, | 30 | -9000 |
| | | | R(3, 4, 27, 28)→N/Q | | |
| RDW/FA4**iv** | 6-24 | **11-22** | N(1,30)→R, R(27,28)→N/Q | 34/33 | -9500 / -8800 |
| RDWA5**i** | 10-17 | 7-**25** | - | 41 | -9200 |
| RDW/FA5**ii** | 10-17 | **11-18** | - | 44/37 | -9300 / -9100 |
| RDWA6**i** | 10-20 | **11-22** | - | 37 | -9900 |
| RDWA7**i** | 10-21 | **7-25** | - | 32 | -9400 |
| RDW/FA8**i** | 10-24 | **11-22** | - | 25/32 | -9400 / -9300 |

| **Group B** | | | | | |
|---|---|---|---|---|---|
| RDWB12**i** | 6-24 | **11-22** | N/Q(1, 2, 29, 30)→R, | 31 | -10800 |
| | | | R(3, 4, 27, 28)→N/Q | | |
| RDW/FB13**i** | 10-17 | 11-**18** | - | 35/32 | -10500 / -10000 |

From the simulations with the preformed membrane pore, the inventors identified the positions in the sequence suitable for stable intermolecular SB interactions. Overall, the stability of SB pairs followed the trend 6-17 ≈ 6-21 < 6-24 < 6-20 for the interactions employing R6 and 10-20 ≈ 10-24 < 10-21 < 10-17 employing R10 on the first peptide-peptide interface (Table 5). Similarly, the trend was 7-18 < 7-22 < 7-25 employing R7 and 11-25 < 11-18 < 11-22 employing R11 on the second interface (Table 5). These trends were more pronounced in groups C and D, where two SB pairs were tested in all possible combinations on each interface, compared to groups A and B, where a single SB pair was tested on each interface. Note that the charged residues at positions 10 and 21 were able to form SBs not only on the designed interface, but at both the interfaces. Despite of the formation of these additional 7-21, 10-22, and 11-21 intermolecular SBs (Table 5), the pores remained stable till 51 µs. Further, three additional SBs 3-20, 3-24, and 3-25 between the oppositely charged residues from two neighboring transmembrane peptide termini were identified (Table 6). These SBs also contributed to the pore stabilization.

**Table 5. Time-averaged numbers and the standard deviations of the formed salt bridge (SB) interactions in pores stabilized by the peptides from Table 3 and 4 investigated in the simulations with preformed pore in POPC and POPE:POPG (3:1 mol/mol) membranes.**

| **Intermolecular interactions** | | **Additional interactions** | |
|---|---|---|---|
| **First peptide-peptide interface** | **Second peptide-peptide interface** | **Intermolecular** | **Intramolecular** |
| 6-17 SB: 4.5 ± 0.8 | 7-18 SB: 1.3 ± 0.4 | 7-21 SB: 3.2 ± 1.2 | 11-18 SB: 3.1 ± 0.7 |
| 6-20 SB: 5.4 ± 0.3 | 7-22 SB: 5.5 ± 1.0 | 10-22 SB: 2.6 ± 1.0 | 14-17 SB: 5.2 ± 1.2 |
| 6-21 SB: 4.6 ± 0.5 | 7-25 SB: 6.4 ± 0.6 | 11-21 SB: 4.5 ± 1.0 | 14-18 SB: 6.4 ± 1.0 |
| 6-24 SB: 5.4 ± 0.9 | | | |
| | 11-18 SB: 6.4 ± 0.9 | | |
| 10-17 SB: 7.5 ± 0.2 | 11-22 SB: 6.6 ± 1.0 | | |
| 10-20 SB: 3.5 ± 0.9 | 11-25 SB: 3.0 ± 0.5 | | |
| 10-21 SB: 6.5 ± 1.0 | | | |
| 10-24 SB: 4.7 ± 0.6 | | | |
| | | | |
| 14-17 SB: 4.6 ± 1.9 | | | |

Other than the intended intermolecular interactions, the inventors also discovered the unintended formation of intramolecular SBs. Firstly, in the middle of the sequence, four oppositely charged residues from two intended SBs (14-17 and 11-18) organized in a closely packed quadrupole arrangement on each interface. In this configuration, both inter- and intramolecular SBs were identified simultaneously (Table 5) and resulted in the decreased pore stability for RDWA9 (7 transmembrane peptides in pore after 51 µs). Secondly, at the peptide C-terminus, positively charged terminal residues R27, R28, and R29 formed intramolecular SBs with the negatively charged residues D24 and D25 (Table 6). The stability of designed intermolecular 6-24 and 7-25 SBs improved upon removing those positively charged terminal residues (compare RDFA2 with RDFA2i/ii/iii, and similarly, RDFA4 with RDFA4i/ii/iii in Table 6).

**Table 6. Time-averaged numbers of inter- and intramolecular salt bridge (SB) interactions in pores stabilized by the peptides RDW/FA2 and RDW/FA4 (Table 3) and their mutants (Table 4) investigated in the simulations with preformed pore in POPC membrane.**

| **Peptides** | **Designed intermolecular SBs** | | | | | **Additional SBs at the peptide termini** | | | | | | | |
|---|---|---|---|---|---|---|---|---|---|---|---|---|---|
| | | | | | | **Intermolecular** | | | | | **Intramolecular** | | |
| | **6-20** | **6-24** | **7-22** | **7-25** | **11-22** | **2-20** | **2-24** | **3-20** | **3-24** | **3-25** | **27-24** | **28-25** | **29-25** |
| RDWA2 | 5.1 | - | - | 5.6 | - | - | - | 1.0 | - | 0.6 | - | 5.7 | - |
| RDFA2 | 5.5 | - | - | 7.2 | - | - | - | 1.1 | - | 1.6 | - | 5.8 | - |
| RDFA2i | 5.6 | - | - | 6.5 | - | - | - | 1.0 | - | 2.8 | - | - | - |
| RDFA2ii | 6.0 | - | - | 6.6 | - | - | - | 1.1 | - | 1.5 | - | - | - |
| RDFA2iii | 4.3 | - | - | 0.9 | - | 0.2 | - | - | - | - | - | - | 7.1 |
| RDWA2iv | 5.6 | - | 5.4 | - | - | - | - | 1.1 | - | - | - | - | - |
| RDFA2iv | 5.7 | - | 5.2 | - | - | - | - | 1.1 | - | - | - | - | - |
| RDWA2v | 5.5 | - | - | - | 7.4 | - | - | 2.2 | - | - | - | - | - |
| RDFA2v | 5.0 | - | - | - | 6.3 | - | - | 1.3 | - | - | - | - | - |
| RDWA4 | - | 4.4 | - | 6.7 | - | - | - | - | 1.5 | 0.5 | 5.4 | 6.0 | - |
| RDFA4 | - | 4.2 | - | 6.9 | - | - | - | - | 1.8 | 0.7 | 6.0 | 5.8 | - |
| RDFA4i | - | 6.7 | - | 6.2 | - | - | - | - | 3.9 | 3.4 | - | - | - |
| RDFA4ii | - | 6.2 | - | 6.7 | - | - | - | - | - | - | - | - | - |
| RDFA4iii | - | 5.7 | - | 2.4 | - | - | 0.7 | - | - | - | - | - | 6.5 |
| RDWA4iv | - | 6.1 | - | - | 6.5 | - | - | - | 3.2 | - | - | - | - |
| RDFA4iv | - | 5.1 | - | - | 6.6 | - | - | - | 3.2 | - | - | - | - |

The use of SB interactions in barrel-stave pore stabilization was tested further by switching the positions of two oppositely charged residues forming a SB pair, i.e., D-R instead of R-D (Table 7). As templates, the peptides RDFC16 and RDWC21 were selected to investigate all the designed SB pairs with switched positions of charged residues. Both peptides with switched SBs (i.e., DRFC16 and DRWC21) were able to stabilize pores in both POPC and POPE:POPG (3:1 mol/mol) membranes identical to the template peptides. The stability of SB interactions also did not change significantly with the only exceptions of 10-20 and 10-24 SBs, which gained strength upon switching the positions of residues.

**Table 7. Time-averaged numbers of salt bridge (SB) interactions in pores stabilized by the peptides RDFC16 and RDWC21 (Table 3) and their variants with switched positions of the two oppositely charged residues forming a SB pair (i.e., D-R instead of R-D) investigated in the simulations with preformed pore in POPC membrane. Average number of water molecules inside the pore and peptide-peptide interaction energies were calculated over the last 3 µs (i.e., 48-51 µs) and for the entire 51 µs simulation, respectively.**

| **Peptides** | **Designed SBs** | | | | | | | | | | | | **Water inside pore (Avg.)** | **P-P int. energy (Avg. in kJ/mol)** |
|---|---|---|---|---|---|---|---|---|---|---|---|---|---|---|
| | **First interface** | | | | | | **Second interface** | | | | | | | |
| | **6-17** | **6-20** | **6-21** | **6-24** | **10-17** | **10-20** | **10-21** | **10-24** | **7-22** | **7-25** | **11-22** | **11-25** | | |
| RDFC16 | 1.4 | 5.5 | - | - | 7.6 | 1.4 | - | - | 6.0 | 5.5 | 7.8 | 1.2 | 37 | -10000 |
| DRFC16 | 1.7 | 7.0 | - | - | 7.5 | 5.7 | - | - | 5.6 | 5.5 | 7.9 | 2.2 | 35 | -9900 |
| RDWC21 | - | - | 4.0 | 6.4 | - | - | 7.6 | 2.8 | 7.4 | 7.3 | 7.8 | 1.1 | 20 | -11000 |
| DRWC21 | - | - | 1.6 | 6.2 | - | - | 7.5 | 5.0 | 6.4 | 4.9 | 7.4 | 0.7 | 22 | -10800 |

In general, introducing interfacial SB networks improved the overall pore stability. It was found that the peptides from groups C and D made pores more stable compared to the ones from groups A and B. Subsequently, the inventors validated these findings with the 'scaled' Martini force field (see Materials and Methods for details), because the original version of Martini was shown to overestimate the protein-protein interactions (Javanainen et al. PLOS ONE 2017, 12, 11, e0187936). Peptides designed with four SBs per interface (i.e., groups C and D) retained the pore stability till 51 µs and the designed interactions in the pore remained unaltered. In contrast, only some of the peptides designed with two SBs per peptide-peptide interface were able to stabilize pores till 51 µs (Table 8). These peptides were RDW/FA2 and RDW/FA5 which had the most stable intermolecular SBs 6-20, 7-25, 10-17, and 11-22 in the sequence. Other peptides from group A did not stabilize pores enough as the number of interactions decreased significantly. For example, intramolecular SBs 27-24 and 28-25 hindered intermolecular SBs 6-24 and 7-25, respectively, and resulted in the limited pore stability for RDW/FA4. Scaled Martini also revealed that not only the total strength of attraction between the peptides, but also the balance of interactions between the neighboring interfaces is important for the stability of pores made of two SBs per interface (i.e., groups A and B). Additional intermolecular SBs 7-21, 10-22, and 11-21 induced an imbalance of interactions between the neighboring interfaces and subsequently, resulted in reduced pore stability as seen for RDW/FA3 and A7. Introducing four SBs per interface in groups C and D helped to retain the balance between the neighboring interfaces leading to increased pore stability.

**Table 8. Time-averaged numbers of intermolecular salt bridge (SB) interactions in pores stabilized by the peptides from group A (Table 3) investigated in both standard (non-scaled) and 'scaled' Martini simulations with preformed pore in POPC membrane. TMP stands for the number of transmembrane peptides at the end of simulation.**

| **Peptides** | **Designed SBs (standard / scaled Martini)** | | | | | | | | **Additional SBs (standard / scaled Martini)** | | | **TMP** |
|---|---|---|---|---|---|---|---|---|---|---|---|---|
| | **First interface** | | | | | **Second interface** | | | | | | |
| | **6-20** | **6-21** | **6-24** | **10-17** | **10-21** | **7-22** | **7-25** | **11-22** | **7-21** | **10-22** | **11-21** | |
| RDWA2 | 5.1/5.3 | - | - | - | - | - | 5.6/5.7 | - | - | - | - | 8/8 |
| RDFA2 | 5.5/5.5 | - | - | - | - | - | 7.2/6.7 | - | - | - | - | 8/8 |
| RDWA3 | - | 5.1/0.6 | - | - | - | 7.1/1.1 | - | - | 4.0/0.7 | - | - | 8/0 |
| RDFA3 | - | 4. 1/0.7 | - | - | - | 5.5/1.6 | - | - | 4.1/1.7 | - | - | 8/0 |
| RDWA4 | - | - | 4.4/0.7 | - | - | - | 6.7/1.6 | - | - | - | - | 8/0 |
| RDFA4 | - | - | 4.2/2.5 | - | - | - | 6.9/4.8 | - | - | - | - | 8/6 |
| RDWA5 | - | - | - | 7.3/7.2 | - | - | - | 7.3/6.4 | - | 2.3/0.5 | - | 8/8 |
| RDFA5 | - | - | - | 7.7/7.2 | - | - | - | 7.1/6.3 | - | 2.3/0.7 | - | 8/7 |
| RDWA7 | - | - | - | - | 5.4/1.0 | - | - | 7.5/3.3 | - | 2.4/1.5 | 3.2/0.3 | 8/0 |
| RDFA7 | - | - | - | - | 7.2/3.4 | - | - | 6.8/4.9 | - | 3.3/1.0 | 4.7/2.3 | 8/5 |

A representative octameric barrel-stave pore structure stabilized by the studied RDFA2 peptides in POPC membrane is shown in Figure 3. There were eight transmembrane antiparallel peptides surrounding a continuous water channel of diameter approximately 2.1 nm. Amphipathic peptides oriented themselves in a cuboid shape with the hydrophobic residues interacting with the lipids tails and hydrophilic side chains facing the water channel. By comparing the pores formed by the different model peptides, it was found that the numbers of water molecules inside the pore is inversely proportional to the peptide-peptide interaction energies. As the total number of interactions (i.e., ST + SB) increased in groups A < B < C < D, the pore structure became more compact narrowing down the water channel (Table 3). Moreover, the pores were narrower with RDFs compared to RDW peptides (Table 3 and 4). The stability of pore structures in POPC membrane was virtually indistinguishable from the pores in POPE:POPG (3:1 mol/mol) membrane. In a stable barrel-stave pore, the peptide termini were identified to be in close contact with the lipid head groups (Figure 3). The interaction between the positively charged peptide termini and the lipid head groups was found particularly strong for the negatively charged POPG lipids. Moreover, the electrostatic attraction between the net negatively charged POPE:POPG membrane and the cationic peptides also resulted in stronger peptide-membrane affinity (∼ 5 kcal/mol) compared to the zwitterionic POPC membrane (Table 9).

**Table 9. Free energy of peptide (from Table 3) adsorption (in kJ/mol) on POPC and POPE:POPG membrane surfaces from the umbrella sampling simulations.**

| **Peptides** | **POPC only** | **POPE:POPG (3:1 mol/mol)** |
|---|---|---|
| RDFA2 | 64.4 ± 0.4 | 84.1 ± 2.1 |
| RDFC16 | 63.6 ± 3.3 | 87.0 ± 1.7 |
| RDWC21 | 84.1 ± 7.1 | 105.0 ± 1.3 |
| RDFC21 | 82.0 ± 4.6 | 107.5 ± 2.9 |

Apart from the pore-forming ability, an ideal AMP should also be selective to the bacterial membrane in order to minimize its toxicity against human cells. Peptide net charge has been suggested to be a key factor enhancing selectivity because the outer leaflet of bacterial membrane is mostly anionic, whereas the outer leaflet of human plasma membrane is usually zwitterionic. Furthermore, arginine-to-lysine (R-to-K) substitution was shown to improve the selectivity because K interacts weakly with the zwitterionic, but strongly with the anionic phospholipids. Therefore, the inventors investigated the influence of increased peptide net charge and R-to-K substitutions on the pore-stabilizing ability in 20 model peptides (Table 10), which were designed based on the peptides from Table 3 and 4. The peptide label is extended with +N, denoting the peptide net charge. K denotes the substitution of all arginine residues in the sequence to lysines (e.g., RDFA2 to KDFA2). Kₜₑᵣ denotes the substitution of terminal arginine residues to lysines (i.e., R3, R4, R27, and R28).

**Table 10. Peptides designed (based on the template peptides from Table 3 and 4) to investigate the effects of increased net charge and R-to-K substitutions. All peptides were studied in the simulations with preformed pore inPOPC membrane and additionally, inPOPE:POPG (3:1 mol/mol) membrane for RDWC16Kₜₑᵣ and RDFCI6Kₜₑᵣ. Average number of water molecules inside the pores and peptide-peptide interaction energies were calculated over the last 3 µs (i.e., 48-51 µs) and for the entire 51 µs simulation, respectively.**

| **Peptides** | **Investigated mutations** | **Increase in peptide net charge** | **Water inside pore (Average)** | **P-P int. energy (Average in kJ/mol)** |
|---|---|---|---|---|
| **Group A** | | | | |
| KDFA2 | R→K | - | 46 | -9200 |
| RDFA2i+**8** | Q/N(1,2,10,11,30)→R & R28→Q | 4 | 46 | -9200 |
| KDFA2i+**8** | Q/N(1,2,10,11,30)→K, R28→Q & R→K | 4 | 46 | -9600 |
| RDF A2i+9(**Q18R**) | Q/N(1,2,10,11,18,30)→R & R28→Q | 5 | 40 | -9300 |
| RDF A2ii+**7** | Q/N(1,2,10,11)→R & R28→N | 3 | 46 | -9100 |
| RDWA5+**11** | Q/N(1,2,6,7,24,29,30)→R | 7 | 42 | -10000 |
| RDFA5+**11** | Q/N(1,2,6,7,24,29,30)→R | 7 | 51 | -9500 |
| **K**DFA5+**11** | Q/N(1,2,6,7,24,29,30)→K & R→K | 7 | 38 | -10000 |
| RDFA7+**10** | Q/N(1,2,6,7,29,30)→R | 6 | 34 | -9600 |
| **K**DFA7+**10** | Q/N(1,2,6,7,29,30)→K & R→K | 6 | 31 | -10000 |

| **Group B** | | | | |
|---|---|---|---|---|
| **K**DFB12 | N(1,30)→K, R(27,28)→N/Q & R→K | - | 24 | -10200 |

| **Group C** | | | | |
|---|---|---|---|---|
| RDWC16**Kₜₑᵣ** | R(3,4,27,28)→K | - | 37 | -10500 |
| RDFC16**Kₜₑᵣ** | R(3,4,27,28)→K | - | 33 | -10200 |
| **K**DFC16+**8** | Q/N(1,2,29,30)→K & R→K | 4 | 34 | -10700 |
| **K**DFC17+**8** | Q/N(1,2,29,30)→K & R→K | 4 | 27 | -10300 |
| **K**DFC18+**8** | Q/N(1,2,29,30)→K & R→K | 4 | 25 | -10900 |
| **K**DFC19+**8** | Q/N(1,2,29,30)→K & R→K | 4 | 31 | -10400 |
| **K**DFC20+**8** | Q/N(1,2,29,30)→K & R→K | 4 | 35 | -10700 |
| **K**DFC21+**8** | Q/N(1,2,29,30)→K & R→K | 4 | 22 | -10800 |

| **Group D** | | | | |
|---|---|---|---|---|
| **K**DFD24+**8** | Q/N(1,2,29,30)→K & R→K | 4 | 18 | -11700 |

It was found that the stability of SB interactions in pores remained independent of the nature of positively charged side chains within MARTINI model (Table 11). Introducing positive charges at the peptide termini (i.e., positions 1, 2, 29, and 30) resulted in the formation of new intermolecular (2-20, 2-24, 3-20, 3-24 and 3-25) and intramolecular (27-24, 28-25, and 29-25) SBs involving the charged termini (Table 12). Nevertheless, pores remained stable due to the presence of enough peptide-peptide interactions on each interface (Table 13). Further addition of positive charges on the polar face of the peptides also resulted in the formation of new intermolecular SBs, which reduced the stability of intended interactions, but retained the overall pore stability (Table 14). For example, introducing Rs at positions (6 and 7) and (10, 11 and 18) led to the formation of new SBs (6-17, 6-21 and 7-22) and (10-20, 11-25 and 18-25) in pores formed by the variants of RDFA2 peptides and RDW/FA5 and RDFA7 peptides, respectively.

**Table 11. Time-averaged numbers of intermolecular salt bridge (SB) interactions in pores stabilized by RDW/F peptides (Table 3) and their K variants (i.e., R-to-K substitutions only) (Table 10) investigated in the simulations with preformed pore in POPC membrane.**

| **Peptides** | **Designed SBs** | | | | | | | | | | | **Additional SBs** | | |
|---|---|---|---|---|---|---|---|---|---|---|---|---|---|---|
| | **First interface** | | | | | | | **Second interface** | | | | | | |
| | **6-17** | **6-20** | **6-21** | **6-24** | **10-17** | **10-20** | **10-21** | **7-22** | **7-25** | **11-22** | **11-25** | **7-21** | **10-22** | **11-21** |
| **Group A** | | | | | | | | | | | | | | |
| RDFA2 | - | 5.5 | - | - | - | - | - | - | 7.2 | - | - | - | - | - |
| **K**DFA2 | - | 5.5 | - | - | - | - | - | - | 7.2 | - | - | - | - | - |
| RDFA2i+8 | - | 3.4 | - | - | - | 6.3 | - | - | 2.6 | - | 3.6 | - | - | - |
| **K**DFA2i+8 | - | 4.8 | - | - | - | 4.3 | - | - | 5.9 | - | 5.3 | - | - | - |
| RDFA5+11 | 5.2 | - | - | - | 5.8 | - | - | 4.2 | - | 5.9 | - | - | 1.0 | - |
| **K**DFA5+11 | 7.0 | - | - | - | 7.0 | - | - | 6.8 | - | 7.6 | - | - | 2.6 | - |
| RDFA7+10 | - | - | 3.1 | - | - | - | 5.3 | 4.5 | - | 6.6 | - | 1.3 | 1.6 | 3.2 |
| **K**DFA7+10 | - | - | 3.8 | - | - | - | 6.3 | 5.5 | - | 7.5 | - | 0.8 | 2.9 | 4.1 |

| **Group B** | | | | | | | | | | | | | | |
|---|---|---|---|---|---|---|---|---|---|---|---|---|---|---|
| RDFB 12 | - | - | - | 6.3 | - | - | - | - | 7.7 | - | - | - | - | - |
| **K**DFB12 | - | - | - | 6.3 | - | - | - | - | 7.3 | - | - | - | - | - |

| **Group C** | | | | | | | | | | | | | | |
|---|---|---|---|---|---|---|---|---|---|---|---|---|---|---|
| RDWC16 | 4.4 | 5.8 | - | - | 7.4 | 2.7 | - | 4.7 | 5.4 | 7.7 | 2.4 | - | 3.1 | - |
| RDWC16**Kₜₑᵣ** | 5.1 | 6.5 | - | - | 7.7 | 2.1 | - | 5.4 | 6.0 | 7.8 | 1.7 | - | 3.7 | - |
| RDFC16 | 1.4 | 5.5 | - | - | 7.6 | 1.4 | - | 6.0 | 5.5 | 7.8 | 1.2 | - | 3.7 | - |
| RDFC16**Kₜₑᵣ** | 1.9 | 6.2 | - | - | 7.7 | 1.6 | - | 6.8 | 5.4 | 7.8 | 1.0 | - | 4.1 | - |

**Table 12. Time-averaged numbers of inter- and intramolecular salt bridge (SB) interactions involving the charged terminal residues in pores stabilized by RDW/F peptides (Table 3) and their K variants (i.e., R-to-K substitutions) and addition K residues at the peptide termini (i.e., positions 1, 2, 29, and 30) (Table 10) investigated in the simulations with preformed pore in POPC membrane.**

| **Peptides** | **SB interactions at the peptide termini** | | | | | | | |
|---|---|---|---|---|---|---|---|---|
| | **Intermolecular** | | | | | **Intramolecular** | | |
| | **2-20** | **2-24** | **3-20** | **3-24** | **3-25** | **27-24** | **28-25** | **29-25** |
| **Group C** | | | | | | | | |
| RDFC16 | - | - | 2.9 | - | 0.7 | - | 5.0 | - |
| **K**DFC16+**8** | 2.8 | - | 4.5 | - | 2.0 | - | 6.8 | 5.8 |
| RDFC17 | - | - | - | - | 4.3 | - | 4.6 | - |
| **K**DFC17+**8** | - | - | - | - | 1.6 | - | 6.3 | 6.6 |
| RDFC18 | - | - | - | 2.5 | 1.1 | 6.0 | 5.4 | - |
| **K**DFC18+**8** | - | 1.8 | - | 4.3 | 1.6 | 6.9 | 6.8 | 5.5 |
| RDFC19 | - | - | 1.6 | - | 1.1 | - | 5.0 | - |
| **K**DFC19+**8** | 1.2 | - | 2.3 | - | 1.2 | - | 6.5 | 6.7 |
| RDFC20 | - | - | 1.5 | 2.0 | 1.5 | 6.4 | 6.0 | - |
| **K**DFC20+**8** | 1.4 | 0.8 | 2.5 | 2.4 | 2.2 | 7.2 | 7.1 | 5.7 |
| RDFC21 | - | - | - | 4.7 | 2.9 | 6.2 | 4.7 | - |
| **K**DFC21+**8** | - | 4.4 | - | 4.6 | 1.8 | 6.9 | 6.1 | 6.3 |

| **Group D** | | | | | | | | |
|---|---|---|---|---|---|---|---|---|
| RDFD24 | - | - | - | 2.8 | 0.6 | 6.7 | 5.6 | - |
| **K**DFD24+**8** | - | 3.5 | - | 3.2 | 0.7 | 7.1 | 6.6 | 6.0 |

**Table 13. Time-averaged numbers of intermolecular salt bridge (SB) interactions in pores stabilized by RDW/F peptides (Table 3) and their K variants (i.e., R-to-K substitutions) with Ks introduced additionally at the peptide termini (i.e., positions 1, 2, 29, and 30) (Table 10) investigated in the simulations with preformed pore in POPC membrane.**

| **Peptides** | **Designed SBs** | | | | | | | | | | | | **Additional SBs** | | |
|---|---|---|---|---|---|---|---|---|---|---|---|---|---|---|---|
| | **First interface** | | | | | | | | **Second interface** | | | | | | |
| | **6-17** | **6-20** | **6-21** | **6-24** | **10-17** | **10-20** | **10-21** | **10-24** | **7-22** | **7-25** | **11-22** | **11-25** | **7-21** | **10-22** | **11-21** |
| **Group C** | | | | | | | | | | | | | | | |
| RDFC16 | 1.4 | 5.5 | - | - | 7.6 | 1.4 | - | - | 6.0 | 5.5 | 7.8 | 1.2 | - | 3.7 | - |
| **K**DFC16+**8** | 6.6 | 6.2 | - | - | 6.9 | 1.4 | - | - | 6.6 | 5.9 | 7.2 | 0.4 | - | 4.5 | - |
| RDFC17 | 4.6 | - | 6.2 | - | 7.3 | - | 5.2 | - | 7.5 | 7.3 | 7.6 | 0.4 | 4.9 | 3.0 | 3.9 |
| **K**DFC17+**8** | 5.6 | - | 3.7 | - | 6.1 | - | 3.9 | - | 5.7 | 4.7 | 6.1 | 0.3 | 2.0 | 2.9 | 3.4 |
| RDFC18 | 3.5 | - | - | 3.9 | 7.7 | - | - | 1.7 | 5.8 | 5.5 | 7.7 | 2.1 | - | 3.2 | - |
| **K**DFC18+**8** | 5.8 | - | - | 2.8 | 7.9 | - | - | 0.6 | 6.0 | 6.4 | 7.9 | 0.6 | - | 4.4 | - |
| RDFC19 | - | 5.6 | 2.1 | - | - | 3.9 | 5.1 | - | 6.6 | 5.9 | 7.6 | 0.9 | 2.6 | 3.0 | 4.8 |
| **K**DFC19+**8** | - | 5.4 | 1.2 | - | - | 3.8 | 4.5 | - | 5.1 | 4.9 | 7.8 | 0.4 | 1.2 | 4.3 | 4.4 |
| RDFC20 | - | 5.7 | - | 2.5 | - | 4.1 | - | 2.1 | 5.9 | 6.1 | 7.1 | 1.7 | - | 3.5 | - |
| **K**DFC20+**8** | - | 5.7 | - | 1.3 | - | 4.2 | - | 1.1 | 5.1 | 5.7 | 7.6 | 1.2 | - | 3.7 | - |
| RDFC21 | - | - | 4.7 | 6.6 | - | - | 7.7 | 1.7 | 7.6 | 6.8 | 7.6 | 0.6 | 4.1 | 4.1 | 3.8 |
| **K**DFC21+**8** | - | - | 5.8 | 6.3 | - | - | 6.7 | 1.1 | 6.2 | 5.7 | 7.7 | 0.4 | 1.4 | 4.0 | 3.6 |
| **Group D** | | | | | | | | | | | | | | | |
| RDFD24 | - | - | 3.4 | 5.4 | - | - | 6.7 | 3.0 | 7.5 | 7.4 | 7.9 | 1.0 | 3.2 | 5.5 | 6.0 |
| **K**DFD24+**8** | - | - | 4.2 | 6.5 | - | - | 6.6 | 3.2 | 6.4 | 6.0 | 7.6 | 0.3 | 0.7 | 4.3 | 5.1 |

**Table 14. Time-averaged numbers of intermolecular salt bridge (SB) interactions in pores stabilized by RDW/F peptides (Table 3 and 4) and their mutants with additional Rs introduced at the polar face of the peptides (Table 10) investigated in the simulations with preformed pore in POPC membrane.**

| **Peptides** | **Designed SBs** | | | | | | | | | | **Additional SBs** | | | |
|---|---|---|---|---|---|---|---|---|---|---|---|---|---|---|
| | **First interface** | | | | | | **Second interface** | | | | | | | |
| | **6-17** | **6-20** | **6-21** | **10-17** | **10-20** | **10-21** | **7-22** | **7-25** | **11-22** | **11-25** | **7-21** | **10-22** | **11-21** | **18-25** |
| **Group A** | | | | | | | | | | | | | | |
| RDFA2i | - | 5.6 | - | - | - | - | - | 6.5 | - | - | - | - | - | - |
| RDFA2i+**8** | - | 3.4 | - | - | 6.3 | - | - | 2.6 | - | 3.6 | - | - | - | - |
| RDFA2i+**9(Q18R)** | - | 1.2 | - | - | 6.9 | - | - | 0.9 | - | 1.5 | - | - | - | 5.4 |
| RDFA2ii | - | 6.0 | - | - | - | - | - | 6.6 | - | - | - | - | - | - |
| RDFA2ii+7 | - | 2.5 | - | - | 5.9 | - | - | 2.5 | - | 3.6 | - | - | - | - |
| RDWA5 | - | - | - | 7.3 | - | - | - | - | 7.3 | - | - | 2.3 | - | - |
| RDWA5**+11** | 4.4 | - | - | 5.8 | - | - | 5.0 | - | 6.3 | - | - | 1.4 | - | - |
| RDFA5 | - | - | - | 7.7 | - | - | - | - | 7.1 | - | - | 2.3 | - | - |
| RDFA5+**11** | 5.2 | - | - | 5.8 | - | - | 4.2 | - | 5.9 | - | - | 1.0 | - | - |
| RDFA7 | - | - | - | - | - | 7.2 | - | - | 6.8 | - | - | 3.3 | 4.7 | - |
| RDFA7**+10** | - | - | 3.1 | - | - | 5.3 | 4.5 | - | 6.6 | - | 1.3 | 1.6 | 3.2 | - |

The effects of K substitutions and increased net charge (by mutating polar residues) on peptide's pore-stabilizing ability were tested further with the peptides designed with switched positions of two oppositely charged residues forming a SB pair (i.e., DRFC16 and DRWC21). Additional Ks at the peptide termini (i.e., positions 1, 2, 29, and 30) along with R-to-K substitutions enhanced the stability of 11-25 SB, which depleted the interactions involving position 7, while the other SBs remained unaffected (Table 15). Moreover, the formation of new intermolecular (28-6, 29-7, 29-10 and 29-11) and intramolecular (2-6, 3-6, 3-7 and 4-7) SBs was discovered to involve the charged residues at the termini (Table 16), although the overall pore stability remained without significant change, probably due to sufficient peptide-peptide interactions on each interface. The above results showed that the increase in peptide net charge and R-to-K substitutions can be incorporated to enhance the membrane selectivity of the peptides without affecting the peptides' pore-stabilizing ability.

**Table 15. Time-averaged numbers of salt bridge (SB) interactions in pores stabilized by the peptides DRFC16 and DRWC21 with switched positions of the two oppositely charged residues forming a SB pair (Table 7) and their K variants (i.e., R-to-K substitutions) with Ks introduced additionally at the peptide termini (i.e., positions 1, 2, 29, and 30) investigated in the simulations with preformed pore in POPC membrane. Average number of water molecules inside the pore and peptide-peptide interaction energies were calculated over the last 3 µs (i.e., 48-51 µs) and for the entire 51 µs simulation, respectively.**

| **Peptides** | **Designed SBs** | | | | | | | | | | | | **Water inside pore (Avg.)** | **P-P int. ener. (Avg. in kJ/mol)** |
|---|---|---|---|---|---|---|---|---|---|---|---|---|---|---|
| | **First interface** | | | | | | | **Second interface** | | | | | | |
| | **6-17** | **6-20** | **6-21** | **6-24** | **10-17** | **10-20** | **10-21** | **10-24** | **7-22** | **7-25** | **11-22** | **11-25** | | |
| DRFC16 | 1.7 | 7.0 | - | - | 7.5 | 5.7 | - | - | 5.6 | 5.5 | 7.9 | 2.2 | 35 | -9900 |
| DKFC16+8 | 0.4 | 7.4 | - | - | 7.8 | 7.1 | - | - | 0.7 | 1.1 | 7.7 | 6.0 | 44 | -10500 |
| DRWC21 | - | - | 1.6 | 6.2 | - | - | 7.5 | 5.0 | 6.4 | 4.9 | 7.4 | 0.7 | 22 | -10800 |
| DKWC21+8 | - | - | 0.2 | 6.8 | - | - | 6.5 | 6.8 | 1.4 | 1.3 | 6.0 | 3.8 | 29 | -10900 |

**Table 16. Time-averaged numbers of salt bridge (SB) interactions involving the charged termini in pores stabilized by DRFC16 and DRWC21 peptides and their K variants (i.e., R-to-K substitutions) with Ks introduced additionally at the peptide termini (i.e., positions 1, 2, 29, and 30) (Table 15) investigated in the simulations with preformed pore in POPC membrane.**

| **Peptides** | **SB interactions at the peptide termini** | | | | | | | | |
|---|---|---|---|---|---|---|---|---|---|
| | **Intermolecular** | | | | | **Intramolecular** | | | |
| | **27-6** | **28-6** | **29-7** | **29-10** | **29-11** | **2-6** | **3-6** | **3-7** | **4-7** |
| DRFC16 | 0.4 | 0.9 | - | - | - | - | 6.5 | 7.0 | 5.2 |
| DKFC16+8 | 0.2 | 0.3 | 7.6 | 5.8 | 6.9 | 6.7 | 7.0 | 7.6 | 7.0 |
| DRWC21 | 0.6 | 4.3 | - | - | - | - | 5.8 | 6.3 | 3.3 |
| DKWC21+8 | 0.6 | 4.9 | 5.7 | 2.9 | 5.2 | 7.1 | 6.7 | 7.3 | 6.7 |

Next, the inventors explored the possibilities to stabilize barrel-stave pores without ST interactions by mutating the aromatic (W/F) to aliphatic (L/I) residues . They simulated six peptides to test the effects of either two or four SBs per interface using both the standard (non-scaled) and 'scaled' Martini force fields (Table 17). All the obtained pore structures and the intended SBs were stable in the standard Martini simulations including the peptides designed with only two SBs per interface. However, in the 'scaled' Martini simulations, the pores started to deform for the peptides designed with two SBs per interface. Notably, the pores formed by four SBs per interface remained stable irrespective of the type of the employed force field.

**Table 17. Time-averaged numbers of intermolecular salt bridge (SB) interactions in pores stabilized by RDF peptides (Table 3 and 4) and their RD variants designed without ST interactions (i.e., W/F → L/I substitutions) investigated with both standard (non-scaled) and 'scaled' Martini force fields in the simulations with preformed pore in POPC membrane. Average number of water molecules inside the pore and peptide-peptide interaction energies were calculated over the last 3 µs (i.e., 48-51 µs) and for the entire 51 µs simulation, respectively. TMP stands for the number of transmembrane peptides at the end of simulation.**

| **Peptides** | **Martini force field used** | **Designed SBs** | | | | | | | | | | | | **TMP** | **Water inside pore (Avg.)** | **P-P int. ener. (Avg. in kJ/mol)** |
|---|---|---|---|---|---|---|---|---|---|---|---|---|---|---|---|---|
| | | **First interface** | | | | | | | | **Second interface** | | | | | | |
| | | **6-17** | **6-20** | **6-21** | **6-24** | **10-17** | **10-20** | **10-21** | **10-24** | **7-22** | **7-25** | **11-22** | **11-25** | | | |
| RDFA2i | Standard | - | 5.6 | - | - | - | - | - | - | - | 6.5 | - | - | 8 | 47 | -9000 |
| RD2i | Standard | - | 5.9 | - | - | - | - | - | - | - | 7.0 | - | - | 8 | 42 | -8300 |
| | Scaled | - | 2.1 | - | - | - | - | - | - | - | 1.5 | - | - | 4 | 30 | -6300 |
| RDFA4i | Standard | - | - | - | 6.7 | - | - | - | - | - | 6.2 | - | - | 8 | 28 | -9500 |
| RD4i | Standard | - | - | - | 7.1 | - | - | - | - | - | 6.6 | - | - | 8 | 25 | -8600 |
| | Scaled | - | - | - | 1.9 | - | - | - | - | - | 2.1 | - | - | 3 | 25 | -6400 |
| RDFA5 | Standard | - | - | - | - | 7.7 | - | - | - | - | - | 7.1 | - | 8 | 36 | -8900 |
| RD5 | Standard | - | - | - | - | 7.5 | - | - | - | - | - | 7.6 | - | 8 | 29 | -8200 |
| | Scaled | - | - | - | - | 2.4 | - | - | - | - | - | 3.6 | - | 5 | 30 | -6500 |
| RDFA7 | Standard | - | - | - | - | - | - | 7.2 | - | - | - | 6.8 | - | 8 | 25 | -9500 |
| RD7 | Standard | - | - | - | - | - | - | 5.3 | - | - | - | 7.7 | - | 8 | 25 | -8400 |
| | Scaled | - | - | - | - | - | - | 1.9 | - | - | - | 4.4 | - | 6 | 32 | -6700 |
| RDFC16 | Standard | 1.4 | 5.5 | | | 7.6 | 1.4 | | | 6.0 | 5.5 | 7.8 | 1.2 | 8 | 37 | -9900 |
| RD16 | Standard | 6.3 | 7.0 | - | - | 6.8 | 1.3 | - | - | 7.0 | 6.9 | 7.7 | 0.6 | 8 | 28 | -9500 |
| | Scaled | 6.2 | 7.0 | - | - | 6.7 | 1.4 | - | - | 7.4 | 7.3 | 7.7 | 0.8 | 8 | 34 | -8200 |
| RDFC21 | Standard | | | 4.7 | 6.6 | | | 7.7 | 1.7 | 7.6 | 6.8 | 7.6 | 0.6 | 8 | 23 | -10800 |
| RD21 | Standard | - | - | 4.1 | 5.5 | - | - | 6.5 | 2.5 | 5.6 | 5.5 | 7.7 | 0.7 | 8 | 23 | -9300 |
| | Scaled | - | - | 3.4 | 4.8 | - | - | 5.9 | 2.3 | 5.8 | 5.9 | 7.7 | 0.7 | 8 | 30 | -8000 |

All the above simulated peptides were based on the initial amphipathic pattern LIQN, carrying high amphipathicity. The inventors subsequently investigated the influence of this initial amphipathic character on the pore-stabilizing ability of peptides by substituting the hydrophilic and hydrophobic residues to serine (S) and valine (V), respectively, in at least one peptide from each group A, B, C, and D (Table 18). They observed that the pore stability and the intended interactions reduced significantly for the peptides from groups A and B, leading to noticeably narrower water channels. In contrast, the pores formed by the peptides from groups C and D remained intact with continuous water channels identical to the parent peptides.
The above results demonstrated that the barrel-stave pores can be stabilized even without ST interactions in the presence of four SBs per interface because of stronger peptide-peptide interactions on each interface, which keep the pore stable. The same behavior was obtained for peptides with reduced amphipathicity (SV mutant).

**Table 18. Time-averaged numbers of intermolecular salt bridge (SB) interactions in pores stabilized by K/RDF(+X) peptides (Table 10) and their SV variants (where the polar (Q/N) and non-polar (L/I) patches were mutated to serine (S) and valine (V), respectively) investigated in the simulations with preformed pore in POPC membrane. Average number of water molecules inside the pore and peptide-peptide interaction energies were calculated over the last 3 µs (i.e., 48-51 µs) and for the entire 51 µs simulation, respectively.**

| **Peptides** | **Designed SBs** | | | | | | | | | | | | **Water inside pore (Avg.)** | **P-P int. ener. (Avg. in kJ/mol)** |
|---|---|---|---|---|---|---|---|---|---|---|---|---|---|---|
| | **First interface** | | | | | | | | **Second interface** | | | | | |
| | **6-17** | **6-20** | **6-21** | **6-24** | **10-17** | **10-20** | **10-21** | **10-24** | **7-22** | **7-25** | **11-22** | **11-25** | | |
| **Group A** | | | | | | | | | | | | | | |
| KDFA2 | - | 5.5 | - | - | - | - | - | - | - | 7.2 | - | - | 46 | -9200 |
| KDFA2**SV** | - | 2.2 | - | - | - | - | - | - | - | 2.5 | - | - | 18 | -10300 |
| RDFA2i+**8** | - | 3.4 | - | - | - | 6.3 | - | - | - | 2.6 | - | 3.6 | 46 | -9200 |
| RDFA2i+8**SV** | - | 3.4 | - | - | - | 5.9 | - | - | - | 3.7 | - | 0.5 | 20 | -10900 |

| **Group B** | | | | | | | | | | | | | | |
|---|---|---|---|---|---|---|---|---|---|---|---|---|---|---|
| KDFB 12 | - | - | - | 6.3 | - | - | - | - | - | 7.3 | - | - | 24 | -10200 |
| KDFB 12**SV** | - | - | - | 6.4 | - | - | - | - | - | 4.6 | - | - | 5 | -12000 |

| **Group C** | | | | | | | | | | | | | | |
|---|---|---|---|---|---|---|---|---|---|---|---|---|---|---|
| **K**DFC16+**8** | 6.6 | 6.2 | - | - | 6.9 | 1.4 | - | - | 6.6 | 5.9 | 7.2 | 0.4 | 34 | -10700 |
| KDFC16+8**SV** | 4.5 | 5.9 | - | - | 6.9 | 4.0 | - | - | 5.4 | 5.2 | 7.2 | 1.8 | 29 | -11300 |

| **Group D** | | | | | | | | | | | | | | |
|---|---|---|---|---|---|---|---|---|---|---|---|---|---|---|
| **K**DFD24+**8** | - | - | 4.2 | 6.5 | - | - | 6.6 | 3.2 | 6.4 | 6.0 | 7.6 | 0.3 | 18 | -11700 |
| KDFD24+8**SV** | - | - | 2.5 | 5.9 | - | - | 6.8 | 3.7 | 4.6 | 4.5 | 7.6 | 1.5 | 25 | -12000 |

Furthermore, the inventors investigated the ability of the designed peptides to form barrel-stave pore spontaneously (Table 19). Spontaneous pore formation was found to depend on the initial arrangement of peptides on the membrane. Simulations started from the configurations where the peptides were placed above the membrane, resulted in the formation of protein aggregates either in solution or on the membrane without any transmembrane pore formation. However, the simulations started from the configurations where the peptides were inserted at the lipid glycerol region, led to spontaneous pore formation. The observed sequence of events was: [1] initial insertion of a single peptide from the surface adsorbed state to the transmembrane state, [2] insertion of other peptides into the transmembrane state, associated with membrane deformation and the insertion of water molecules into the membrane core, [3] formation of disordered pore with a continuous water channel, and [4] system relaxation into a regular barrel-stave pore. The pore formation was identified to be a stochastic process as the same initial system configuration with different random seeds resulted in pore formation only in one out of three simulations. Moreover, not all the simulations with the initial insertion of a single peptide into the transmembrane state resulted in the pore formation within the simulation timescale. Note, that the free energy barrier associated with the membrane defects and pore formation could be unrealistically high in Martini force field. After a small reduction of this barrier (see Materials and Methods for details), the inventors observed more frequent events of pore formation.

**Table 19. Spontaneous pore formation by the designed peptides from Table 3. Results are indicated by the number of peptides in the transmembrane state (TMP) at the end of simulation run. Three initial arrangements of peptides on the membrane are as follows- H. higher; peptides in solution immediately above the lipid heads, I: intermediate; peptides at the lipid head-water interface, L: lower; peptides at the lipid head-tail interface, i.e., glycerol region. Symbol * indicates the application of inverted flat bottom potential to the lipid tails for additional 9 µs simulation (see Materials and Methods for details).**

| **Peptides** | **Simulation properties** | **System A:** 8 peptides on each membrane leaflet | | | **System B:** 12 peptides on each membrane leaflet | | | **System** C: 16 peptides on each membrane leaflet | | |
|---|---|---|---|---|---|---|---|---|---|---|
| | | **(I)** | **(II)** | **(III)** | **(I)** | **(II)** | **(III)** | **(I)** | **(II)** | **(III)** |
| **POPC:POPG (1:1 mol/mol) membrane** | | | | | | | | | | |
| RDFA2 | Arrangements | H | I | L | | | | | | |
| | Runtime | 30 µs | 30 µs | 51 µs | | | | | | |
| | TMP | 0 | 0 | 11 | | | | | | |

| **POPE:POPG (3:1 mol/mol) membrane** | | | | | | | | | | |
|---|---|---|---|---|---|---|---|---|---|---|
| RDFA2 | Arrangements | H | I | L | | | | | | |
| | Runtime | 30 µs | 30 µs | 30 µs | | | | | | |
| | TMP | 0 | 0 | 1 | | | | | | |

| **Pure POPC membrane** | | | | | | | | | | |
|---|---|---|---|---|---|---|---|---|---|---|
| RDFC16 | Arrangements | L | L | L | L | L | L | L | L | L |
| | Runtime | 51 µs | 30 µs | 39 µs* | 9 µs | 30 µs | 30 µs | 9 µs | 30 µs | 30 µs |
| | TMP | 14 | 0 | 13 | 0 | 0 | 0 | 0 | 0 | 0 |
| RDWC19 | Arrangements | L | L | L | L | L | L | L | L | L |
| | Runtime | 39 µs* | 30 µs | 30 µs | 30 µs | 30 µs | 30 µs | 30 µs | 24 µs | 30 µs |
| | TMP | 9 | 0 | 0 | 0 | 0 | 0 | 18 | 0 | 0 |

Based on the above findings from MD simulations, the inventors developed a set of rules guiding the design of helical peptides capable of forming stable, antiparallel barrel-stave pores. They formulated 5 design rules, based on which total 52 sequence patterns, organized in four groups (i.e., A, B, C and D), can be generated (Table 20). The rules are as follows:
[1] Peptides have a length of 30 amino acids with a secondary amphiphilic character stretched over 22 residues in the middle of the sequence, i.e., having a continuous hydrophobic patch in α-helical conformation.
[2] Polar amino acids forming the hydrophilic patch could be T, Q, S, or N. Non-polar amino acids forming the hydrophobic patch could be A, M, V, L, or I. Peptides made of residues with higher polarity (i.e., Q and N) and hydrophobicity (i.e., L and I), i.e., higher amphipathicity, are preferred for pore stability. Peptides made of residues with lower polarity and hydrophobicity require further compensation via additional stronger interactions for equal pore stabilization.
[3] Charged residues within the hydrophilic patch are designed to form pore-stabilizing salt bridge (SB) interactions. Either two (group A and B) or four (group C and D) SBs can be designed per peptide-peptide interface. Pores designed with two SBs per interface are less stable and require further stabilization via aromatic residues. Pairs of positions at which the oppositely charged amino acids (R or K with D or E) form SBs are as follows - group A: 6-20 or 10-17 on the first interface, and 7-25 or 11-22 on the second interface; group B: 6-24 or 10-17 on the first interface, and 7-25 or 11-22 on the second interface; group C and D: the first pair out of 6-17, 6-20, 6-21, or 6-24, and the second pair out of 10-17, 10-20, 10-21 or 10-24 on the first interface, and the first pair out of 7-22 or 7-25, and the second pair out of 11-22 or 11-25 on the second interface. Note that the above SB pairs should be combined without duplication. Overall, the stability of SB pairs increases as follows: 10-20 ≈ 10-24 < 6-17 ≈ 6-21 < 6-24 < 6-20 < 10-21 < 10-17 on the first interface, and 11-25 < 7-22 < 7-25 < 11-22 on the second interface.
[4] Aromatic non-polar amino acids (W and F) provide additional pore stabilization via π-π stacking (ST) interactions within the hydrophobic patch. The preferred positions are 13 and 15 for one ST per peptide-peptide interface (group A and C), and 8, 9, 20, and 26 for two STs per interface (group B and D). ST interactions are necessary for designing peptides with two SBs per interface (group A and B).
[5] Four residues at each peptide terminus should be either polar or positively charged. These additional basic residues could enhance the peptide selectivity to bacterial membranes. But the possibilities of intramolecular SB interactions should be avoided while designing peptides with two SBs per interface (group A and B). Therefore, the oppositely charged amino acids should not be combined at the following positions - 6, 7, 24 and 25, with 2 or 3, 3 or 4, 27, and 28 or 29, respectively.

**Table 20. Sequence patterns of length 30 residues (with a hydrophobic patch stretched over 22 residues) designed to stabilize antiparallel barrel-stave pores. Sequences are categorized into four groups, namely A, B, C and D, based on the total number of intermolecular stacking (ST) and salt bridge (SB) interactions which can be designed on each peptide-peptide interface within pore. The patterns are represented with the sequence identifiers, where p indicates uncharged polar amino acids (T, Q, S, or N); h indicates non-polar hydrophobic amino acids (A, M, V, L, or I); b and a represent basic (R or K) and acidic (D or E) amino acids, respectively, which form SBs (or can be replaced by p); s represents aromatic non-polar amino acids (W or F), which forms STs (or can be replaced by h); and t represents terminal residues (which is either p or b).**

| **Sr. No.** | **Proposed SB interactions** | | **Proposed sequence patterns** |
|---|---|---|---|
| | **First peptide-peptide interface** | **Second peptide-peptide interface** | |
| **Group** A: One 13-13 ST (interface 1) or one 15-15 ST (interface 2) and two SBs per interface | | | |
| 1 | 6-20 | 7-25 | tttthbbhhpphspshpphapphpahtppt (SEQ ID NO. 121) |
| 2 | | | tttthbbhhbbhspshpphapphpahtppt (SEQ ID NO. 122) |
| 3 | | | tppphaahhpphspshpphbpphpbhtttt (SEQ ID NO. 123) |
| 4 | | | tppphaahhpphspshbphbpbhpbhtttt (SEQ ID NO. 124) |
| 5 | 6-20 | 11-22 | tttthbphhpbhspshpphapahpphtttt (SEQ ID NO. 125) |
| 6 | | | tttthbbhhbbhspshpphapahpphtttt (SEQ ID NO. 126) |
| 7 | | | tppthaphhpahspshpphbpbhpphtttt (SEQ ID NO. 127) |
| 8 | | | tppthaphhpahspshbphbpbhpbhtttt (SEQ ID NO. 128) |
| 9 | 10-17 | 7-25 | tttthpbhhbphspshaphhpphpahtppt (SEQ ID NO. 129) |
| 10 | | | tttthbbhhbbhspshaphhpphpahtppt (SEQ ID NO. 130) |
| 11 | | | ttpphpahhaphspshbphhpphpbhtttt (SEQ ID NO. 131) |
| 12 | | | ttpphpahhaphspshbphbpbhpbhtttt (SEQ ID NO. 132) |
| 13 | 10-17 | 11-22 | tttthpphhbbhspshaphhpahpphtttt (SEQ ID NO. 133) |
| 14 | | | tttthbbhhbbhspshaphhpahpphtttt (SEQ ID NO. 134) |
| 15 | | | tttthpphhaahspshbphhpbhpphtttt (SEQ ID NO. 135) |
| 16 | | | tttthpphhaahspshbphbpbhpbhtttt (SEQ ID NO. 136) |

| **Group B:** Two 9-20 STs (interface 1) or two 8-26 STs (interface 2) and two SBs per interface | | | |
|---|---|---|---|
| 17 | 6-24 | 7-25 | tttthbbsspphhphhpphspphaaspppt (SEQ ID NO. 137) |
| 18 | | | |
| 19 | | | tttthbbssbbhhphhpphspphaaspppt (SEQ ID NO. 138) |
| 20 | | | tppphaasspphhphhpphspphbbstttt (SEQ ID NO. 139) |
| | | | tppphaasspphhphhbphspbhbbstttt (SEQ ID NO. 140) |
| 21 | 6-24 | 11-22 | tttthbpsspbhhphhpphspahapspttt (SEQ ID NO. 141) |
| 22 | | | tttthbbssbbhhphhpphspahapspttt (SEQ ID NO. 142) |
| 23 | | | tppthapsspahhphhpphspbhbpstttt (SEQ ID NO. 143) |
| 24 | | | tppthapsspahhphhbphspbhbbstttt (SEQ ID NO. 144) |
| 25 | 10-17 | 7-25 | tttthpbssbphhphhaphspphpastppt (SEQ ID NO. 145) |
| 26 | | | tttthbbssbbhhphhaphspphpastppt (SEQ ID NO. 146) |
| 27 | | | ttpphpassaphhphhbphspphpbstttt (SEQ ID NO. 147) |
| 28 | | | ttpphpassaphhphhbphspbhbbstttt (SEQ ID NO. 148) |
| 29 | 10-17 | 11-22 | tttthppssbbhhphhaphspahppstttt (SEQ ID NO. 149) |
| 30 | | | tttthbbssbbhhphhaphspahppstttt (SEQ ID NO. 150) |
| 31 | | | tttthppssaahhphhbphspbhppstttt (SEQ ID NO. 151) |
| 32 | | | tttthppssaahhphhbphspbhbbstttt (SEQ ID NO. 152) |

| **Group C:** One 13-13 ST (interface 1) or one 15-15 ST (interface 2) and four SBs per interface | | | |
|---|---|---|---|
| 33 | 6-17 & 10-20 \| 6-20 & 10-17 | 7-22 & 11-25 \| 7-25 & 11-22 | tttthbbhhbbhspshaphapahpahtttt (SEQ ID NO. 153) |
| 34 | | | tttthaahhaahspshbphbpbhpbhtttt (SEQ ID NO. 154) |
| 35 | 6-17 & 10-21 \| 6-21 & 10-17 | | tttthbbhhbbhspshaphhaahpahtttt (SEQ ID NO. 155) |
| 36 | | | tttthaahhaahspshbphhbbhpbhtttt (SEQ ID NO. 156) |
| 37 | 6-17 & 10-24 \| 6-24 & 10-17 | | tttthbbhhbbhspshaphhpahaahtttt (SEQ ID NO. 157) |
| 38 | | | tttthaahhaahspshbphhpbhbbhtttt (SEQ ID NO. 158) |
| 39 | 6-20 & 10-21 \| 6-21 & 10-20 | | tttthbbhhbbhspshpphaaahpahtttt (SEQ ID NO. 159) |
| 40 | | | tttthaahhaahspshpphbbbhpbhtttt (SEQ ID NO. 160) |
| 41 | 6-20 & 10-24 \| 6-24 & 10-20 | | tttthbbhhbbhspshpphapahaahtttt (SEQ ID NO. 161) |
| 42 | | | tttthaahhaahspshpphbpbhbbhtttt (SEQ ID NO. 162) |
| 43 | 6-21 & 10-24 \| 6-24 & 10-21 | | tttthbbhhbbhspshpphhaahaahtttt (SEQ ID NO. 163) |
| 44 | | | tttthaahhaahspshpphhbbhbbhtttt (SEQ ID NO. 164) |
| 45 | All inclusive | | tttthaahhaahspshbphbbbhbbhtttt (SEQ ID NO. 165) |

| **Group D:** Two 9-20 STs (interface 1) or two 8-26 STs (interface 2) and four SBs per interface | | | |
|---|---|---|---|
| 46 | 6-17 & 10-21 \| 6-21 & 10-17 | 7-22 & 11-25 \| 7-25 & 11-22 | tttthbbssbbhhphhaphsaahpastttt (SEQ ID NO. 166) |
| 47 | | | tttthaassaahhphhbphsbbhpbstttt (SEQ ID NO. 167) |
| 48 | 6-17 & 10-24 \| 6-24 & 10-17 | | tttthbbssbbhhphhaphspahaastttt (SEQ ID NO. 168) |
| 49 | | | tttthaassaahhphhbphspbhbbstttt (SEQ ID NO. 169) |
| 50 | 6-21 & 10-24 \| 6-24 & 10-21 | | tttthbbssbbhhphhpphsaahaastttt (SEQ ID NO. 170) |
| 51 | | | tttthaassaahhphhpphsbbhbbstttt (SEQ ID NO. 171) |
| 52 | All inclusive | | tttthaassaahhphhbphsbbhbbstttt (SEQ ID NO. 172) |

To validate the simulation-guided rules to design antimicrobial peptides able to stabilize barrel-stave pores, the designed peptides were tested *in vitro* for their abilities to induce vesicle leakage (pore-forming activity), kill bacteria (antimicrobial activity), and toxicity (Table 21). Firstly, the experiments were performed with 21 representative peptides which were able to stabilize preformed membrane pores in simulation. Based on the findings, ten additional peptides were derived from the most promising peptide candidate, KDFA2i+9-NH2, to further increase its antimicrobial activity. Since, amidation of peptide C-terminus has been shown to improve antimicrobial activity, the effects of C-terminus amidation was also tested with the designed peptides. All the sequences can be found in Table 23. The ability of the designed peptides to form leaky transmembrane pores was demonstrated by the peptide-induced leakage of fluorescent dye calcein from the large unilamellar vesicles (LUVs). Representative peptides from each of the four designed groups A, B, C and D resulted in a concentrationdependent calcein leakage from POPC:POPG (1:1 mol/mol) LUVs, which served as a simplified mimic of bacterial membrane. The extent of leakage was above 50% and 100% at peptide-to-lipid (P:L) ratios 1:100 and 1:20, respectively, for 10 out of 13 vesicle-disrupting peptides. The 8 peptides, which did not cause calcein leakage, had solubilization problem, and formed visible aggregates in PBS buffer. The only exception was KDFC16+9SV-NH2 peptide, which had its hydrophilic and hydrophobic patches formed by S and V residues, respectively. In general, the peptides made of K residues caused more calcein leakage than the corresponding R variants (RDFA2 versus KDFA2 and RDFA2i+9-NH2 versus KDFA2i+9-NH2). The inventors found that pore-forming ability of KDFA9 was retained even after placing the oppositely charged residues at positions allowing intramolecular SBs. Further, group C peptide KDFC16+9-NH2 without ST (i.e., KD16+9-NH2) and with switched SBs (i.e., DKFC16+9-NH2) also resulted in vesicle leakage, which is in line with our computational findings.

**Table 21. Results of in vitro experiments with the designed peptides - peptide-induced leakage of fluorescent dye calcein from POPC:POPG (1:1 mol/mol) large unilamellar vesicles (LUVs), peptide antimicrobial efficacy against Gram-negative Escherichia coli and Gram-positive Staphylococcus carnosus bacteria and toxicity against human red blood cells (HRBCs) and human dermal fibroblasts (HDFs). MIC stands for minimum inhibitory concentration against bacteria and EC₅₀ is the concentration causing 50% inhibition of the human cell growth. Antibiotic ampicillin and the known hemolytic peptide melittin were used as controls.**

| **Sr. No.** | **Investigated agents** | **Group** | **Calcein leakage assay** | | | | **Broth microdilution assay** | | **Hemolysis assay** | |
|---|---|---|---|---|---|---|---|---|---|---|
| | | | **Extent of leakage (in percentage) at increasing peptide-to-lipid ratio (mol/mol)** | | | | **Antibacterial activity (MIC in µM)** | | **Toxicity (EC50 in µM)** | |
| | | | **1:100** | **1:50** | **1:20** | **1:10** | ***E. coli* TOP10** | ***S*. *carnosus*** | **HRBCs** | **HDFs** |
| 1 | RDWA2 | A | Aggregated peptides and no leakage | | | | - | - | - | - |
| 2 | RDFA2 | A | 80.2 ± 8.2 | 97.4 ± 4.5 | 102.9 ± 0.9 | - | > 50 | - | > 50 | - |
| 3 | KDFA2 | A | 82.8 ± 1.5 | 100.2 ± 0.2 | - | - | 50 | - | > 50 | - |
| 4 | RDFA2i | A | Aggregated peptides and no leakage | | | | - | - | - | - |
| 5 | RDFA2i+9-NH2 | A | 65.8 ± 4.1 | 101.1 ± 0.8 | - | - | 1.6 | 0.8 | 0.9 | 16 |
| | | | | | | | | | | |
| 6 | KDFA2i+9-NH2 | A | 87.1 ± 2.7 | 97.8 ± 1.6 | 102.3 ± 0.7 | - | 0.8 | 0.8 | > 50 | 97 |
| 7 | RDFA5 | A | Aggregated peptides and no leakage | | | | - | - | - | - |
| 8 | KDFA5+11-NH2 | A | 38.6 ± 3.5 | 78.0 ± 4.3 | 101.6 ± 1.4 | | 1.6 | 1.6 | > 50 | - |
| 9 | RDFA7 | A | Aggregated peptides and no leakage | | | | - | - | - | - |
| 10 | KDFA9 | A | 62.6 ± 5.2 | 92.0 ± 0.3 | 102.0 ± 1.4 | - | > 50 | > 50 | > 50 | - |
| 11 | KDFB12 | B | 79.4 ± 3.7 | 98.2 ± 0.7 | 101.5 ± 0.6 | - | 6.3 | 6.3 | > 50 | 20 |
| 12 | RDFC16 | C | Aggregated peptides and no leakage | | | | - | - | - | - |
| 13 | RDFC16Kter | C | 64.5 ± 6.1 | 85.3 ± 4.3 | 102.2 ± 0.5 | - | 50 | - | > 50 | - |
| 14 | KDFC16+9-NH2 | C | 78.2 ± 4.5 | 97.4 ± 9.3 | 98.3 ± 5.2 | 99.28 ± 1.2 | 0.8 | 0.4 | > 50 | 10 |
| 15 | KD16+9-NH2 | C | 58.8 ± 4.8 | 83.0 ± 1.5 | 93.1 ± 0.7 | 99.1 ± 2.5 | 6.3 | 3.1 | > 50 | - |
| 16 | KDFC16+9SV-NH2 | C | No leakage | | | | 25 | 0.4 | > 50 | > 150 |
| 17 | DKFC16+9-NH2 | C | 50.1 ± 4.1 | 89.5 ± 3.3 | 103.3 ± 0.9 | - | > 50 | 1.6 | > 50 | - |
| 18 | RDWC21 | C | Aggregated peptides and no leakage | | | | - | - | - | - |
| 19 | RDFC21 | C | Aggregated peptides and no leakage | | | | - | - | - | - |
| 20 | KDFC21+9-NH2 | C | 5 1.9 ± 1.4 | 78.2 ± 1.2 | 99.4 ± 1.6 | 102.2 ± 1.1 | 3.1 | 3.1 | > 50 | - |
| 21 | KDFD24+9-NH2 | D | 13.0 ± 5.2 | 42.6 ± 11.5 | 81.5 ± 6.8 | 98.7 ± 3.2 | 12.5 | 12.5 | > 50 | - |

| **Optimization of the most promising peptide KDFA2i+9-NH2 for better antimicrobial activity** | | | | | | | | | | |
|---|---|---|---|---|---|---|---|---|---|---|
| 22 | KDWA2i+9-NH2 | A | 29.6 ± 1.2 | 92.0 ± 3.6 | 103.2 ± 0.5 | - | 0.4 | 0.8 | > 50 | - |
| 23 | KEFA2i+9-NH2 | A | 29.2 ± 4.9 | 87.0 ± 3.8 | 101.0 ± 0.4 | - | 0.8 | 0.8 | > 50 | - |
| 24 | RDFA2i+9-NH2-Kter | A | 76.0 ± 3.3 | 100.7 ± 1.0 | - | - | 0.2 | 0.4 | 49 | - |
| 25 | KDFA2i+9-NH2-Rter | A | 65.5 ± 4.2 | 98.8 ± 1.2 | 103.3 ± 1.2 | - | 0.4 | 0.8 | 6.9 | - |
| 26 | KDFA2i+8 | A | 68.0 ± 1.7 | 98.9 ± 2.3 | 103.2 ± 0.3 | - | 0.2 | 0.4 | > 50 | - |
| 27 | KDFA2i+8(I26F) | A | 91.6 ± 2.8 | 98.8 ± 0.8 | 103.1 ± 0.8 | - | 0.4 | 0.8 | > 50 | - |
| 28 | KDFA2i+7-npNH2 | A | Aggregated peptides and no leakage | | | | - | - | - | - |
| 29 | KDFA2i+11-NH2 | A | 93.7 ± 3.1 | 102.9 ± 0.8 | - | - | 0.2 | 0.2 | > 50 | - |
| 30 | KDFA2i+2-Hter | A | 102.5 ± 1.2 | - | - | - | > 50 | > 50 | > 50 | - |
| 31 | KDFA2i+12 | A | 74.0 ± 6.7 | 100.6 ± 1.5 | - | - | 0.4 | 0.4 | > 50 | - |

| **Controls** | | | | | | | | | | |
|---|---|---|---|---|---|---|---|---|---|---|
| 32 | Ampicillin | - | - | | | | 12.5 | 0.1 | - | - |
| 33 | Melittin | - | - | | | | - | | 0.9 | - |

The antibacterial activity of the designed peptides was tested in broth microdilution assay determining the minimum bacterial inhibition concentration (MIC). All the vesicle disrupting peptides, with the exceptions of RDFA2 and KDFA9, showed antimicrobial activity against both Gram-negative *E. coli* and Gram-positive *S*. *carnosus* bacteria. It was found that the increase in peptide net charge correlates with the antibacterial activity. For example, KDFA2 and RDFC16Kter (with net charge +4 e) inhibited *E. coli* at the highest tested concentration of 50 µM, while their highly cationic variants KDFA2i+9-NH2 and KDFC16+9-NH2 (with net charge +9 e), respectively, caused bacterial inhibition at 0.8 µM. The peptides made of K residues were more effective than the corresponding R variants (KDFA2 versus RDFA2 and KDFA2i+9-NH2 versus RDFA2i+9-NH2), which is in line with the previous report (Yang et al. FEBS letters 2003, 540, 1-3, 229-233). Against *E. coli,* peptides KDFA2i+9-NH2 and KDFC16+9-NH2 were the most active ones with MIC 0.8 µM, which is 15 times lower than the MIC of the common antibiotic ampicillin. Against *S*. *carnosus,* peptides KDFC16+9-NH2 and its SV variant KDFC16+9SV-NH2 were the most effective ones with MIC 0.4 µM, which is however 4 times higher than that of ampicillin. Interestingly, although peptide DKFC 16+9-NH2 with reversed SBs did not show activity against *E. coli,* it was found active against *S*. *carnosus* demonstrating bacterial specificity. Note that the designed peptides showed bactericidal rather than the bacteriostatic effect in the nutrient-rich medium.
The toxicity of the designed peptides was evaluated in hemolysis assay against the human red blood cells (HRBCs) and in neutral red uptake assay against the human dermal fibroblasts (HDF). Among all the tested peptides, only RDFA2i+9-NH2 showed hemolytic activity below the highest tested concentration of 50 µM and its half maximal inhibitory concentration (i.e., EC₅₀) was 0.9 µM, which is comparable to that of the known hemolytic peptide melittin. All the investigated peptides showed good fibroblast cell viability at the biologically active concentrations and their cytotoxic concentrations were at least an order of magnitude higher than the MICs. Interestingly, the peptides made of R residues were found more toxic than the corresponding K variants (RDFA2i+9-NH2 versus KDFA2i+9-NH2), which is in agreement with the previous findings (Yang et al. FEBS letters 2003, 540, 1-3, 229-233). The least cytotoxic peptide was KDFC16+9SV-NH2, which did not affect the dermal cell viability even at the highest tested concentration of 150 µM.
Peptide biological activity and toxicity were used to determine the therapeutic index (TI), which evaluates the relative safety of a drug. TI was calculated as the ratio of cytotoxic EC₅₀ values and bacterial MIC values. Our most promising peptide KDFA2i+9-NH2 had TI_{(HRBCs-bacteria)} > 63, TI_{(HDF-bacteria)} ≈ 121, where larger TI indicates greater selectivity of the peptide for bacteria over human cells. Therefore, this peptide was optimized further. The following substitutions were tested experimentally: F-to-W (KDWA2i+9-NH2); D-to-E (KEFA2i+9-NH2); RDFA2i+9-NH2 with R-to-K at both termini (RDFA2i+9-NH2-Kter) and KDFA2i+9-NH2 with K-to-R at both termini (KDFA2i+9-NH2-Rter); negatively charged free C-terminus alone (KDFA2i+8) and with I28-to-F substitution (KDFA2i+8(I26F)); non-polar hydrophobic C-terminus (i.e., K-to-A) (KDFA2i+7-npNH2) and charged C-terminus (i.e., Q/N-to-K) (KDFA2i+11-NH2) with amidation; K-to-H substituted charged termini (KDFA2i+2-Hter) and extra K residues at the termini (i.e., total length = 32 residues) (KDFA2i+12) with negatively charged free C-terminus. Out of all these variants, only the non-polar hydrophobic termini (KDFA2i+7-npNH2) led to peptide aggregation, for which the activity could not be determined. All the other peptides showed improved activity against *E. coli* with the only exception of D-to-E variant. Moreover, four peptides RDFA2i+9-NH2-Kter, KDFA2i+8, KDFA2i+11-NH2 and KDFA2i+12 were found more effective against *S. carnosus.* Only the R-containing peptides showed hemolytic activity below the highest tested concentration of 50 µM, where R termini exhibited more toxicity than the corresponding K termini. Note that H residue is neutral at the experimental pH 7.4, and thus the peptide KDFA2i+2-Hter carries a net charge +2 e. Possibly, due to such lower net charge, this peptide did not show antibacterial activity, even though it caused 100% vesicle leakage already at the starting P:L ratio 1:100. The peptides KDFA2i+8(I26F) and KDFA2i+11-NH2 had leakage activity comparable to the parent peptide, while the others caused lesser vesicle disruption at P:L ratio 1:100.
In addition, 7 promising candidates were tested against the most common multi-drug resistant bacteria, known as ESKAPE pathogens (Table 22), for which new antibiotics are urgently needed. Remarkably, almost all the tested pathogens were found susceptible to the designed peptides within the highest tested concentrations, even though they were mostly resistant to ampicillin. Collectively, the highest activity was obtained against *E. faecium, K. pneumoniae, A. baumannii* and *E. coli,* for which our most promising peptide KDFA2i+11-NH2 had MICs 1.9, 3.8, 1.4, and 1.4 µM, respectively. In contrast, the susceptibility toward ampicillin was > 16, > 16, > 16, and 2 µM, respectively, for the same pathogenic strains. The bactericidal effect was achieved even within 4 minutes of exposure.

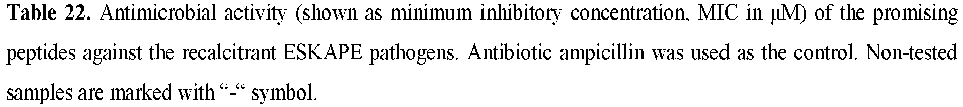

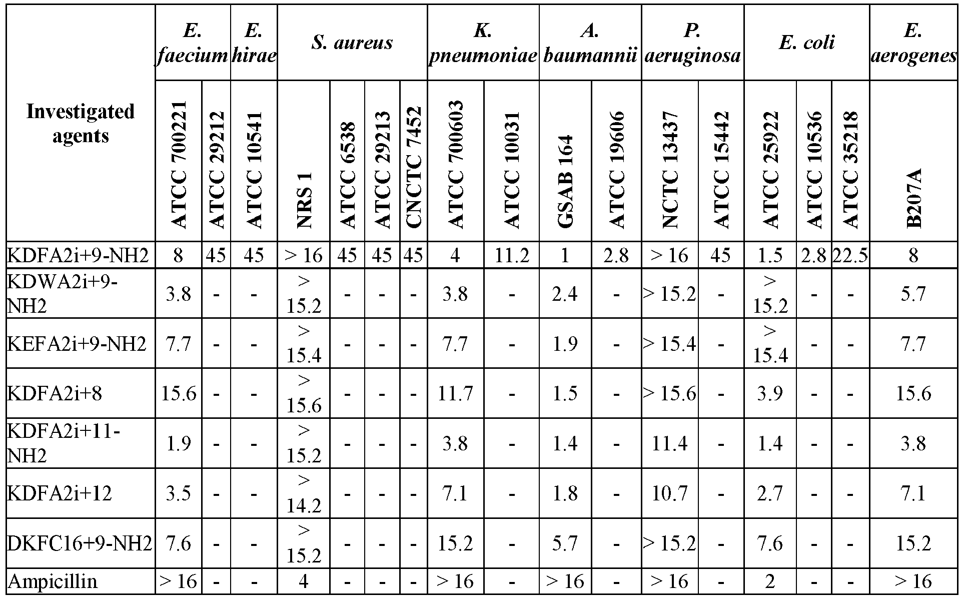

Based on the above *in vitro* experimental results, the inventors reported 36 potent antimicrobial core sequence patterns of length 22 amino acids (SEQ ID NO. 1-36) or more preferably, 36 sequence patterns of length 30 amino acids (SEQ ID NO. 37-72) which are able to form stable barrel-stave pores in the bacterial membranes. It was found that the increase in peptide net charge and R-to-K substitutions led to increased peptide solubility, stronger vesicle leakage, higher antimicrobial activity, and lowered cytotoxicity, which are in excellent agreement with the previous findings. Accordingly, the inventors screened the sequences from Table 20 for the potent sequence patterns concerning antimicrobial activity. Firstly, the sequences with higher net charge were selected because their antibacterial activity was higher than the activity of the sequences with lower net charge. Secondly, the sequences with positively charged termini were selected for the same reason. Peptide C-terminus was proposed to remain either amidated (-CONH₂) or free (-COO⁻), even though the latter showed slightly higher antibacterial activity. Thirdly, due to lower toxicity and higher antimicrobial activity, K residue was selected as the only positively charged basic residue in the sequence. The oppositely charged and aromatic residues were strategically positioned within the sequence to form pore-stabilizing intermolecular salt bridge (SB) and stacking (ST) interactions, respectively. Further, the sequences were categorized into four groups A, B, C, and D based on the total number of SB and ST interactions on each peptide-peptide interface within the pore. The representative peptides from each group were experimentally verified to form leaky transmembrane pores in the bacterial membrane mimicking LUVs and exhibited a broad spectrum of potent bactericidal activity with the MIC as low as 0.2 µM against the common Gram-negative and Gram-positive bacteria, and MIC from 1 µM against the resistant ESKAPE pathogens. Last but not the least, the peptides showed negligible toxicity to the human cells, i.e., providing a wide therapeutic window.

**Table 23. Primary structure of all the investigated peptides. Highlighted sequences were tested in vitro in addition to simulations. All peptides have positively charged N-terminus and negatively charged C-terminus unless it is specified that the C-terminus is amidated (-NH2).**

| **Sr.no.** | **Designed peptides** | **Primary structures** |
|---|---|---|
| **Group A** (one stacking and two salt bridge interactions per peptide-peptide interface) | | |
| 1 | RDWA1 | NQ*RR*L*RR*ILNQLWQWI*DD*LINQLQNI*RR*QN (SEQ ID NO. 173) |
| 2 | RDFA1 | NQ*RR*L*RR*ILNQLFQFI*DD*LINQLQNI*RR*QN (SEQ ID NO. 174) |
| 3 | RDWA1i | NQ*RR*L*RR*ILNQLWQWI*D*QLIN*D*LQNI*RR*QN (SEQ ID NO. 175) |
| 4 | RDFA1i | NQ*RR*L*RR*ILNQLFQFI*D*QLIN*D*LQNI*RR*QN (SEQ ID NO. 176) |
| 5 | RDWA1ii | NQ*RR*L*R*NILN*R*LWQWI*D*QLIN*D*LQNI*RR*QN (SEQ ID NO. 177) |
| 6 | RDFA1ii | NQ*RR*L*R*NILN*R*LFQFI*D*QLIN*D*LQNI*RR*QN (SEQ ID NO. 178) |
| 7 | RDWA2 | NQ*RR*L*RR*ILNQLWQWINQL*D*NQLQ*D*I*RR*QN (SEQ ID NO. 179) |
| 8 | RDFA2 | NQ*RR*L*RR*ILNQLFQFINQL*D*NQLQ*D*I*RR*QN (SEQ ID NO. 180) |
| 9 | KDFA2 | NQKKLKKILNQLFQFINQL*D*NQLQ*D*I*KK*QN (SEQ ID NO. 181) |
| 10 | KDFA2SV | SS*KK*V*KK*VVSSVFSFVSSV*D*SSVS*D*V*KK*SS (SEQ ID NO. 182) |
| 11 | RDFA2i | NQ*RR*L*RR*ILNQLFQFINQL*D*NQLQ*D*I*R*QN*R* (SEQ ID NO. 183) |
| 12 | RDFA2i+8 | *RRRR*L*RR*IL*RR*LFQFINQL*D*NQLQ*D*I*R*QN*R* (SEQ ID NO. 184) |
| 13 | RDFA2i+9-NH2 | *RRRR*L*RR*IL*RR*LFQFINQL*D*NQLQ*D*I*R*QN*R*-NH2 (SEQ ID NO. 185) |
| 14 | RDFA2i+9-NH2-Kter | *KKKK*L*RR*IL*RR*LFQFINQL*D*NQLQ*D*I*K*QN*K*-NH2 (SEQ ID NO. 186) |
| 15 | KDFA2i+9-NH2-Rter | *RRRR*L*KK*IL*KK*LFQFINQL*D*NQLQ*D*I*R*QN*R*-NH2 (SEQ ID NO. 187) |
| 16 | RDFA2i+8SV | *RRRR*V*RR*VV*RR*VFSFVSSV*D*SSVS*D*V*R*SS*R* (SEQ ID NO. 188) |
| 17 | KDFA2i+9-NH2 | KKKKLKKILKKLFQFINQLDNQLQDIKQNK-NH2 (SEQ ID NO. 83) |
| 18 | KDWA2i+9-NH2 | KKKKLKKILKKLWQWINQLDNQLQDIKQNK-NH2 (SEQ ID NO. 84) |
| 19 | KEFA2i+9-NH2 | *KKKK*L*KK*IL*KK*LFQFINQL*E*NQLQ*E*I*K*QN*K*-NH2 (SEQ ID NO. 85) |
| 20 | KDFA2i+8 | *KKKK*L*KK*IL*KK*LFQFINQL*D*NQLQ*D*I*K*QN*K* (SEQ ID NO. 189) |
| 21 | KDFA2i+8 (I26F) | *KKKK*L*KK*IL*KK*LFQFINQL*D*NQLQ*D*F*K*QN*K* (SEQ ID NO. 86) |
| 22 | KDFA2i+7-npNH2 | KKKKLKKILKKLFQFINQLDNQLQDIAQNA-NH2 (SEQ ID NO. 190) |
| 23 | KDFA2i+11-NH2 | *KKKK*L*KK*IL*KK*LFQFINQL*D*NQLQ*D*I*KKKK*-NH2 (SEQ ID NO. 87) |
| 24 | KDFA2i+10-Hter | *HHHH*L*KK*IL*KK*LFQFINQL*D*NQLQ*D*I*HHHH* (SEQ ID NO. 191) |
| 25 | KDFA2i+12 | *KKKKK*L*KK*IL*KK*LFQFINQL*D*NQLQ*D*I*KKKKK* (SEQ ID NO. 88) |
| 26 | RDFA2i+9(Q18R) | *RRRR*L*RR*IL*RR*LFQFINRL*D*NQLQ*D*I*R*QN*R* (SEQ ID NO. 192) |
| 27 | RDFA2ii | NQ*RR*L*RR*ILNQLFQFINQL*D*NQLQ*D*I*R*NQN (SEQ ID NO. 193) |
| 28 | RDFA2ii+7 | *RRRR*L*RR*IL*RR*LFQFINQL*D*NQLQ*D*I*R*NQN (SEQ ID NO. 194) |
| 29 | RDFA2iii | *RR*NQL*RR*ILNQLFQFINQL*D*NQLQ*D*INQ*RR* (SEQ ID NO. 195) |
| 30 | RDWA2iv | NQ*RR*L*RR*ILNQLWQWINQL*D*N*D*LQNI*RR*QN (SEQ ID NO. 196) |
| 31 | RDFA2iv | NQ*RR*L*RR*ILNQLFQFINQL*D*N*D*LQNI*RR*QN (SEQ ID NO. 197) |
| 32 | RDWA2v | NQ*RR*L*RR*ILN*R*LWQWINQL*D*N*D*LQNI*RR*QN (SEQ ID NO. 198) |
| 33 | RDFA2v | NQ*RR*L*R*NILN*R*LFQFINQL*D*N*D*LQNI*RR*QN (SEQ ID NO. 199) |
| 34 | RDWA3 | NQ*RR*L*RR*ILNQLWQWINQLI*DD*LQNI*RR*QN (SEQ ID NO. 200) |
| 35 | RDFA3 | NQ*RR*L*RR*ILNQLFQFINQLI*DD*LQNI*RR*QN (SEQ ID NO. 201) |
| 36 | RDWA4 | NQ*RR*L*RR*ILNQLWQWINQLINQL*DD*I*RR*QN (SEQ ID NO. 202) |
| 37 | RDFA4 | NQ*RR*L*RR*ILNQLFQFINQLINQL*DD*I*RR*QN (SEQ ID NO. 203) |
| 38 | RDFA4i | *R*Q*RR*L*RR*ILNQLFQFINQLINQL*DD*INQN*R* (SEQ ID NO. 204) |
| 39 | RDFA4ii | *R*QN*R*L*RR*ILNQLFQFINQLINQL*DD*INQN*R* (SEQ ID NO. 205) |
| 40 | RDFA4iii | *RR*NQL*RR*ILNQLFQFINQLINQL*DD*INQ*RR* (SEQ ID NO. 206) |
| 41 | RDWA4iv | *R*Q*RR*L*R*NILN*R*LWQWINQLIN*D*L*D*NINQN*R* (SEQ ID NO. 207) |
| 42 | RDFA4iv | *R*Q*RR*L*R*NILN*R*LFQFINQLIN*D*L*D*NINQN*R* (SEQ ID NO. 208) |
| 43 | RDWA5 | NQ*RR*LQNIL*RR*LWQWI*D*QLIN*D*LQNI*RR*QN (SEQ ID NO. 209) |
| **44** | **RDFA5** | **NQRRLQNILRRLFQFIDQLINDLQNIRRQN (SEQ ID NO. 210)** |
| **45** | **KDFA5+11-NH2** | ***KKKK*L*KK*IL*KK*LFQFI*D*QLIN*D*LQNI*KKKK*-NH2 (SEQ ID NO.** 211) |
| 46 | RDWA5i | NQ*RR*LQ*R*IL*R*QLWQWI*D*QLINQLQ*D*I*RR*QN (SEQ ID NO. 212) |
| 47 | RDWA5+11 | *RRRR*L*RR*IL*RR*LWQWI*D*QLIN*D*L*R*NI*RRRR* (SEQ ID NO. 213) |
| 48 | RDFA5+11 | *RRRR*L*RR*IL*RR*LFQFI*D*QLIN*D*L*R*NI*RRRR* (SEQ ID NO. 214) |
| 49 | KDFA5+11 | *KKKK*L*KK*IL*KK*LFQFI*D*QLIN*D*L*K*NI*KKKK*(SEQ ID NO. 215) |
| 50 | RDWA6 | NQ*RR*LQNIL*RR*LWQWIN*D*L*D*NQLQNI*RR*QN (SEQ ID NO. 216) |
| 51 | RDFA6 | NQ*RR*LQNIL*RR*LFQFIN*D*L*D*NQLQNI*RR*QN (SEQ ID NO. 217) |
| 52 | RDWA6i | NQ*RR*LQNIL*RR*LWQWINQL*D*N*D*LQNI*RR*QN (SEQ ID NO. 218) |
| 53 | RDWA7 | NQ*RR*LQNIL*RR*LWQWINQLI*DD*LQNI*RR*QN (SEQ ID NO. 219) |
| **54** | **RDFA7** | **NQRRLQNILRRLFQFINQLIDDLQNIRRQN (SEQ ID NO.220)** |
| 55 | RDFA7+10 | *RRRR*L*RR*IL*RR*LFQFINQLI*DD*LQNI*RRRR* (SEQ ID NO. 221) |
| 56 | KDFA7+10 | *KKKK*L*KK*IL*KK*LFQFINQLI*DD*LQNI*KKKK* (SEQ ID NO. 222) |
| 57 | RDWA7i | NQ*RR*LQ*R*IL*R*QLWQWINQLI*D*QLQ*D*I*RR*QN (SEQ ID NO. 223) |
| 58 | RDWA8 | NQ*RR*LQNIL*RR*LWQWINQLINQL*DD*I*RR*QN (SEQ ID NO. 224) |
| 59 | RDFA8 | NQ*RR*LQNIL*RR*LFQFINQLINQL*DD*I*RR*QN (SEQ ID NO. 225) |
| 60 | RDWA8i | NQ*RR*LQNIL*RR*LWQWINQLIN*D*L*D*NI*RR*QN (SEQ ID NO. 226) |
| 61 | RDFA8i | NQ*RR*LQNIL*RR*LFQFINQLIN*D*L*D*NI*RR*QN (SEQ ID NO. 227) |
| 62 | RDWA9 | NQ*RR*LQNILN*R*LWRWI*DD*LINQLQNI*RR*QN (SEQ ID NO. 228) |
| **63** | **KDFA9** | **NQ*KK*LQNILN*K*LFKFI*DD*LINQLQNI*KK*QN (SEQ ID NO.229)** |

| **Group B** (two stacking and two salt bridge interactions per peptide-peptide interface) | | |
|---|---|---|
| 64 | RDWB10 | NQ*RR*L*RR*WWNQLIQLI*D*QLWN*D*LQNW*RR*QN (SEQ ID NO. 230) |
| 65 | RDFB10 | NQ*RR*L*RR*FFNQLIQLI*D*QLFN*D*LQNF*RR*QN (SEQ ID NO. 231) |
| 66 | RDWB11 | NQ*RR*L*RR*WWNQLIQLINQLW*DD*LQNW*RR*QN (SEQ ID NO. 232) |
| 67 | RDFB11 | NQ*RR*L*RR*FFNQLIQLINQLF*DD*LQNF*RR*QN (SEQ ID NO. 233) |
| 68 | RDWB12 | NQ*RR*L*RR*WWNQLIQLINQLWNQL*DD*W*RR*QN (SEQ ID NO. 234) |
| 69 | RDFB12 | NQ*RR*L*RR*FFNQLIQLINQLFNQL*DD*F*RR*QN (SEQ ID NO. 235) |
| **70** | **KDFB12** | ***K*Q*KK*L*KK*FFNQLIQLINQLFNQL*DD*FNQN*K* (SEQ ID NO.236)** |
| 71 | KDFB12SV | *K*S*KK*V*KK*FFSSVVSVVSSVFSSV*DD*FSSS*K* (SEQ ID NO. 237) |
| 72 | RDFB12i | *RR*QNL*R*NWWN*R*LIQLINQLWN*D*L*D*NWNQ*RR* (SEQ ID NO. 238) |
| 73 | RDWB13 | NQ*RR*LQNWW*RR*LIQLI*D*QLWN*D*LQNW*RR*QN (SEQ ID NO. 239) |
| 74 | RDFB13 | NQ*RR*LQNFF*RR*LIQLI*D*QLFN*D*LQNF*RR*QN (SEQ ID NO. 240) |
| 75 | RDWB14 | NQRRLQNWWRRLIQLINQLWDDLQNWRRQN (SEQ ID NO. 241) |
| 76 | RDFB14 | NQ*RR*LQNFF*RR*LIQLINQLF*DD*LQNF*RR*QN (SEQ ID NO. 242) |
| 77 | RDWB15 | NQRRLQNWWRRLIQLINQLWNDLDNWRRQN (SEQ ID NO. 243) |
| 78 | RDFB15 | NQ*RR*LQNFF*RR*LIQLINQLFN*D*L*D*NF*RR*QN (SEQ ID NO. 244) |

| **Group C** (one stacking and four salt bridge interactions per peptide-peptide interface) | | |
|---|---|---|
| 79 | RDWC16 | NQ*RR*L*RR*IL*RR*LWQWI*D*QL*D*N*D*LQ*D*I*RR*QN (SEQ ID NO. 245) |
| **80** | **RDFC16** | **NQ*RR*L*RR*IL*RR*LFQFI*D*QL*D*N*D*LQ*D*I*RR*QN (SEQ ID NO. 246)** |
| 81 | RDWC16Kter | NQ*KK*L*RR*IL*RR*LWQWI*D*QL*D*N*D*LQDI*KK*QN (SEQ ID NO. 247) |
| **82** | **RDFC16Kter** | **NQ*KK*L*RR*IL*RR*LFQFI*D*QL*D*N*D*LQ*D*I*KK*QN (SEQ ID NO. 248)** |
| 83 | KDFC16+8 | *KKKK*L*KK*IL*KK*LFQFI*D*QL*D*N*D*LQ*D*I*KKKK* (SEQ ID NO. 89) |
| **84** | **KDFC16+9-NH2** | ***KKKK*L*KK*IL*KK*LFQFI*D*QL*D*K*D*LQ*D*I*KKKK*-NH2 (SEQ ID NO. 249)** |
| 85 | KDFC16+8SV | *KKKK*V*KK*JV*KK*VFSFV*D*SV*D*S*D*VS*D*V*KKKK* (SEQ ID NO. 250) |
| **86** | **KDFC16+9SV-NH2** | ***KKKK*V*KK*VV*KK*VFSFV*D*SV*D*S*D*VS*DV*KKKK-NH2 (SEQ ID NO. 251)** |
| 87 | DRFC16 | NQ*RR*L*DD*IL*DD*LFQFI*R*QL*R*N*R*LQ*R*I*RR*QN (SEQ ID NO. 252) |
| 88 | DKFC16+8 | *KKKK*L*DD*IL*DD*LFQFI*K*QL*K*N*K*LQ*K*I*KKKK* (SEQ ID NO. 253) |
| **89** | **DKFC16+9-NH2** | ***KKKK*L*DD*IL*DD*LFQFI*K*QL*K*N*K*LQ*K*I*KKKK*-NH2 (SEQ ID NO. 254)** |
| 90 | RDWC17 | NQ*RR*L*RR*IL*RR*LWQWI*D*QLI*DD*LQ*D*I*RR*QN (SEQ ID NO. 255) |
| 91 | RDFC17 | NQ*RR*L*RR*IL*RR*LFQFI*D*QLI*DD*LQ*D*I*RR*QN (SEQ ID NO. 256) |
| 92 | KDFC17+8 | *KKKK*L*KK*IL*KK*LFQFI*D*QLI*DD*LQ*D*I*KKKK* (SEQ ID NO. 257) |
| 93 | RDWC18 | NQ*RR*L*RR*IL*RR*LWQWI*D*QLIN*D*L*DD*I*RR*QN (SEQ ID NO. 258) |
| 94 | RDFC18 | NQ*RR*L*RR*IL*RR*LFQFI*D*QLIN*D*L*DD*I*RR*QN (SEQ ID NO. 259) |
| 95 | KDFC18+8 | *KKKK*L*KK*IL*KK*LFQFI*D*QLIN*D*L*DD*I*KKKK* (SEQ ID NO. 260) |
| 96 | RDWC19 | NQ*RR*L*RR*IL*RR*LWQWINQL*DDD*LQ*D*I*RR*QN (SEQ ID NO. 261) |
| 97 | RDFC19 | NQ*RR*L*RR*IL*RR*LFQFINQL*DDD*LQ*D*I*RR*QN (SEQ ID NO. 262) |
| 98 | KDFC19+8 | *KKKK*L*KK*IL*KK*LFQFINQL*DDD*LQ*D*I*KKKK* (SEQ ID NO. 263) |
| 99 | RDWC20 | NQ*RR*L*RR*IL*RR*LWQWINQL*D*N*D*L*DD*I*RR*QN (SEQ ID NO. 264) |
| 100 | RDFC20 | NQ*RR*L*RR*IL*RR*LFQFINQL*D*N*D*L*DD*I*RR*QN (SEQ ID NO. 265) |
| 101 | KDFC20+8 | *KKKKLKKILKKLFQFINQLDNDLDDIKKKK* (SEQ ID NO. 266) |
| **102** | **RDWC21** | **NQ*RR*L*RR*IL*RR*LWQWINQLI*DD*L*DD*I*RR*QN (SEQ ID NO. 267)** |
| **103** | **RDFC21** | **NQ*RR*L*RR*IL*RR*LFQFINQLI*DD*L*DD*I*RR*QN (SEQ ID NO. 268)** |
| 104 | KDFC21+8 | *KKKK*L*KK*IL*KK*LFQFINQLI*DD*L*DD*I*KKKK* (SEQ ID NO. 269) |
| **105** | **KDFC21+9-NH2** | ***KKKK*L*KK*IL*KK*LFQFINQLI*DD*L*DD*I*KKKK*-NH2 (SEQ ID NO. 270)** |
| 106 | DRWC21 | NQ*RR*L*DD*IL*DD*LWQWINQLI*RR*L*RR*I*RR*QN (SEQ ID NO. 271) |
| 107 | DKWC21+8 | KKKKL*DD*IL*DD*LWQWINQLI*KK*L*KK*I*KKKK* (SEQ ID NO. 272) |

| **Group D** (two stacking and four salt bridge interactions per peptide-peptide interface) | | |
|---|---|---|
| 108 | RDWD22 | NQ*RR*L*RR*WW*RR*LIQLI*D*QLW*DD*LQ*D*W*RR*QN (SEQ ID NO. 273) |
| 109 | RDFD22 | NQ*RR*L*RR*F*FRR*LIQLI*D*QLF*DD*LQDF*RR*QN (SEQ ID NO. 274) |
| 110 | RDWD23 | NQ*RR*L*RR*WW*RR*LIQLI*D*QLWN*D*L*DD*W*RR*QN (SEQ ID NO. 275) |
| 111 | RDFD23 | NQ*RR*L*RR*FF*RR*LIQLI*D*QLFN*D*L*DD*F*RR*QN (SEQ ID NO. 276) |
| 112 | RDWD24 | NQ*RR*L*RR*WW*RR*LIQLINQLW*DD*L*DD*W*RR*QN (SEQ ID NO. 277) |
| 113 | RDFD24 | NQ*RR*L*RR*FF*RR*LIQLINQLF*DD*L*DD*F*RR*QN (SEQ ID NO. 278) |
| 114 | KDFD24+8 | *KKKK*L*KK*FF*KK*LIQLINQLF*DD*L*DD*F*KKKK* (SEQ ID NO. 279) |
| **115** | **KDFD24+9-NH2** | ***KKKK*L*KK*FF*KK*LIQLINQLF*DD*L*DD*F*KKKK*-NH2 (SEQ ID NO. 280)** |
| 116 | KDFD24+8SV | *KKKK*V*KK*FF*KK*VVSVVSSVF*DD*V*DD*F*KKKK* (SEQ ID NO. 281) |

| **Additional** (no stacking, only salt bridge interaction) | | |
|---|---|---|
| 117 | RD2i | NQ*RR*L*RR*ILNQLIQLINQL*D*NQLQ*D*I*R*QN*R* (SEQ ID NO. 282) |
| 118 | RD4i | *R*Q*RR*L*RR*ILNQLIQLINQLINQL*DD*INQN*R* (SEQ ID NO. 283) |
| 119 | RD5 | NQ*RR*LQNIL*RR*LIQLI*D*QLIN*D*LQNI*RR*QN (SEQ ID NO. 284) |
| 120 | RD7 | NQ*RR*LQNIL*RR*LIQLINQLI*DD*LQNI*RR*QN (SEQ ID NO. 285) |
| 121 | RD16 | NQ*RR*L*RR*IL*RR*LIQLI*D*QL*D*N*D*LQ*D*I*RR*QN (SEQ ID NO. 286) |
| **122** | **KD16+9-NH2** | ***KKKK*V*KK*IL*KK*LIQLI*D*QL*D*K*D*LQ*D*I*KKKK*-NH2 (SEQ ID NO. 287)** |
| 123 | RD21 | NQ*RR*L*RR*IL*RR*LIQLINQLI*DD*L*DD*I*RR*QN (SEQ ID NO. 288) |

| | | **Control** |
|---|---|---|
| 124 | **Melittin** | GIGAVLKVLTTGLPALISWIKRKRQQ-NH2 (SEQ ID NO. 289) |

The inventors used computer simulations to demonstrate the role of individual positions in the peptide sequence for the stabilization of transmembrane pore via intermolecular peptide-peptide interactions. Based on the *in silico* results of more than a hundred model peptides, they created a method for designing and producing helical peptide able to stabilize barrel-stave pores. The core sequence patterns having the length of 22 amino acids (SEQ ID NO. 1-36) are amphipathic in nature, i.e., having a continuous hydrophobic patch made of 10-11 amino acids. Peptides are designed to be stabilized in pores in antiparallel transmembrane arrangements via intermolecular salt bridge networks formed between the complementary charged residues within the hydrophilic part of the peptide. Additional pore stabilization is achieved via intermolecular aromatic π-π stacking interactions established between the aromatic residues within the hydrophobic part of the peptide. The core sequences (SEQ ID NO. 1-36) are preferably extended by 4 (or more) residues on each terminus, which aid the selective adsorption of the peptides on the targeted bacterial membranes and resulted in extended sequences (SEQ ID NO. 37-72). The designed peptides were verified to form leaky pores in the large unilamellar vesicles using *in vitro* fluorescent dye leakage assay. Moreover, the tested peptides exhibited potent broad-spectrum bactericidal activity against both Gram-negative and Gram-positive bacteria with MIC as low as 0.2 µM. The peptides are also active against the multidrug-resistant ESKAPE pathogens with MIC as low as 1 uM. Importantly, the peptides showed negligible toxicity to human cells, i.e., offering a wide therapeutic window.

### Materials and Methods

### Computer Simulations

Molecular dynamics simulations were performed in the Gromacs program package (version 5.1.4.) (Abraham et al. SoftwareX 2015, 1-2, 19-25) using coarse grained Martini force field (Marrink et al. J. Phys. Chem. B 2007, 111, 27, 7812-7824; Monticelli et al. J. Chem. Theory Comput. 2008, 4, 5, 819-834). This model is based on four-to-one mapping where approximately every four heavy atoms (non-hydrogens) are represented by a single bead.

The initial atomistic structure of the peptides was constructed in helical conformation using PyMOL (DeLano, W. L. The PyMOL Molecular Graphics System, Version 1.8. Schrödinger LLC. 2002). All-atom structures were then converted to the Martini representations by martinize.py script (De Jong et al. J. Chem. Theory Comput. 2013, 9, 1, 687-697). The secondary structure of the peptides was selected and kept helical during the simulations. Membrane bilayer systems were prepared in xy-plane using the Martini Maker tool (Qi et al. J. Chem. Theory Comput. 2015, 11, 9, 4486-4494) developed in CHARMM-GUI (http://www.charmm-gui.org) (Jo et al. J. Comput. Chem. 2008, 29, 11, 1859-1865). Three types of bilayers were chosen in our simulation studies: the first bilayer contained only 1-Palmitoyl-2-oleoyl-sn-glycero-3-phosphocholine (POPC) lipids, the second bilayer was composed of POPC and 1-Palmitoyl-2-oleoyl-sn-glycero-3-phospho-(1'-rac-glycerol) (POPG) lipid mixture in 1:1 molecular ratio, and the third bilayer was made of 1-Palmitoyl-2-oleoyl-sn-glycero-3-phosphoethanolamine (POPE) and POPG lipid mixture in 3:1 molecular ratio. Respectivly, these bilayers represented a standard zwitterionic membrane and two different simple mimics of the negatively charged bacterial membranes. Addition of peptides in the bilayer system was dependent on the type of simulations and is described in detail below. For all the simulations, the peptide-bilayer system was solvated with approximately 12000 nonpolarizable water beads. Na+ and Cl- ions were added to neutralize the system and to reach the physiological salt concentration of 0.154 M. The constructed system was then subjected to energy minimization using steepest descent method. Subsequently, the system was equilibrated in five consecutive steps with increasing time step and simulation length in NPT ensemble: (1) 25 ps with 1 fs time step, (2) 250 ps with 5 fs time step, (3) 1 ns with 10 fs time step, (4) 5 ns with 20 fs time step and (5) 1 µs with 20 fs time step. Leap-frog algorithm was used for integrating Newton's equations of motion. A time step of 30 fs was used for the production run. The temperature of the system was kept at 320 K using velocity rescale thermostat (Bussi et al. J. Chem. Phys. 2007, 126, 1, 014101) with 1 ps coupling applied separately to protein-membrane and water-ions. Semi-isotropic pressure coupling with a coupling constant of 12 ps and a compressibility value of 3×10-4 bar-1 was applied independently to the membrane plane and to the membrane normal using Parrinello-Rahman barostat (Parrinello et al. J. Appl. Phys. 1981, 52, 12, 7182-7190) set at 1 bar. Periodic boundary conditions were employed. Verlet cutoff scheme (Páll et al. Comput. Phys. Commun. 2013, 184, 12, 2641-2650) was used with a straight cutoff distance of 1.1 nm and the neighbor list update in every 20 steps. Van der Waals and Coulomb interactions were cutoff at 1.1 nm. Coulomb interactions were treated using the reaction field method (Tironi et al. J. Chem. Phys. 1995, 102, 13, 5451-5459) with the relative dielectric constant of 15. Bonds were constrained using LINCS algorithm (Hess et al. J. Comput. Chem. 1997, 18, 12, 1463-1472).

### Three different types of protein-membrane simulations were performed:

**Simulations with preformed membrane pore.** The inventors investigated the ability of our peptides to stabilize the preformed membrane pore formed in POPC and POPE:POPG (3:1 mol/mol) lipid bilayers with the peptide/lipid molecular ratio 8/504. The pore was initially created in the center of an equilibrated bilayer by disfavoring the presence of lipids in a cylinder with the radius of 2 nm using inverted flat bottom potential applied to the lipid tails with a force constant of 1000 kJ mol-1 nm-2. Peptides were then inserted perpendicular to the membrane plane in the antiparallel orientation along the edge of the pore roughly 1 nm apart with their hydrophobic side facing the lipid tails. During the five steps of equilibration, the pore was kept open using inverted flat bottom potential so that the peptides could assume their preferred orientations in pore. After the equilibration the pore constrain was removed. The final production run was performed for 51 µs. The pore was considered closed when more than four peptides left the transmembrane state and no water channel prevailed. The pore was considered deformed when an intermittent water channel surrounded by four or more transmembrane peptides appeared. Lastly, the pore was considered stable when more than five transmembrane peptides retained antiparallelly surrounding a continuous water channel. Because Martini force field has been reported to overestimate the protein-protein interactions (Javanainen et al. PLOS ONE 2017, 12, 11, e0187936), the selected pores were simulated for another 51 µs with the 'scaled' Martini parameters where the peptide-peptide Lennard-Jones interaction was scaled down by 10%.

**Simulations of spontaneous pore formation.** The inventors investigated the ability of our peptides to form spontaneous transmembrane pores in POPC bilayer with three different peptide/lipid molecular ratios 16/504, 24/504, and 32/504. Additional simulations were performed in POPC:POPG (1:1 mol/mol) and POPE:POPG (3:1 mol/mol) bilayers with the peptide/lipid molecular ratio 16/504. Equal number of peptides were placed initially on each membrane leaflet with their hydrophobic side facing the bilayer interior. In the simulations with zwitterionic bilayer, the peptides were placed initially at the lipid head-tail interface (i.e., glycerol region) and three individual simulations were performed for each system with different initial velocities. In the simulations with the negatively charged bilayers, the peptides were placed initially in solution immediately above the lipid heads, at the lipid head-water interface, and at the lipid head-tail interface. The production run was performed for up to 51 µs for the peptides which formed pore spontaneously. Otherwise, the inventors stopped the simulation after 30 µs and applied inverted flat bottom potential (localized within a cylinder of 1 nm radius and with force constant 1000 kJ mol-1 nm-2) to the lipid tails for additional 9 µs simulation investigating the pore formation in the locally thinned membrane.

**Umbrella sampling of peptide adsorption on membrane plane.** The inventors calculated the potential of mean force (PMF) of peptide adsorption on POPC and POPE:POPG (3:1 mol/mol) bilayers using umbrella sampling method (Torrie et al. J. Comput. Phys. 1977, 23, 2, 187-199). The symmetric protein-bilayer system was composed of 240 lipids and two peptides (one peptide per membrane leaflet). The free energy was calculated along the membrane normal (z-axis) between the membrane and the peptide center of mass (CoM). A total pull of 4 nm was performed over 80 ns using a force constant of 2500 kJ mol-1 nm-2 and pull rate of 0.05 nm ns-1. Slower pull rates 0.01 nm ns-1 and 0.03 nm ns-1 were also tested with very similar results within the error. To prevent the lipid desorption during the initial pull, a flat bottom potential was applied on the lipids. The potential was kept zero up to 2.45 nm from the membrane center, beyond which there was a harmonic potential with a force constant of 250 kJ mol-1 nm-2. The starting configurations for the umbrella sampling windows were constructed with 0.1 nm distance between the peptide and the membrane CoM. Each window was simulated for 550 ns.

Analysis. Visual inspection of the simulated configurations was performed using the visual molecular dynamics (VMD) (Humphrey et al. J. Mol. Graph. 1996, 14, 1, 33-38). Number of water beads in pore was calculated within the slab of 2 nm from the membrane CoM. Tilt angle of the transmembrane peptides in pores was calculated as the angle between the peptide helical axis and the membrane normal. Aromatic or charged residues forming stacking or salt bridge interaction pairs, respectively, were considered to be within the interaction contact when their pairwise distance was shorter than 0.8 nm, which is the position of the first minima in their radial distribution functions. Overall, an interaction was considered stable when at least half of the possible contacts were maintained on an average during the whole simulation. Pore diameter was calculated as the approximate length of the side of the square (formed by joining the centrally located Q14 residues) which can fit inside the pore. Analysis of the umbrella sampling simulations was performed using the weighted histogram analysis method (WHAM) (Kumar et al. J. Comput. Chem. 1992, 13, 8, 1011-1021).

### Experiments.

**Chemicals.** The synthesized peptides were purchased from JPT Peptide Technologies GmbH (Berlin, Germany), Apigenex, S.r.o. (Prague, Czech Republic), and Caslo ApS (Kongens Lyngby, Denmark). Phospholipids POPC and POPG were procured from Avanti Polar Lipids (Alabaster, USA). Lipophilic carbocyanine dye DiD (DiIC18(5); 1,1'-dioctadecyl-3,3,3',3'-tetramethylindodicarbocyanine, 4-chlorobenzenesulfonate salt) was acquired from Life Technologies (Eugene, USA). NaH2PO4·H2O, NaCl, NaOH were purchased from Merck (Darmstadt, Germany). Na2HPO4·7H2O, EDTA, Chloroform, Ethanol, Methanol were procured from VWR (Solon, USA). Calcein and Triton X-100 were obtained from Sigma Aldrich (St. Louis, USA). MHB and MHA powder were purchased from Condalab (Madrid, Spain). After storing at -20 °C, the peptides were dissolved in phosphate-buffered saline (PBS). Lipids and DiD were dissolved in chloroform and stored at -20 °C. The bacterial stock was stored at -80°C. The PBS buffer used in leakage assay contained 25 mM NaH₂PO₄ and Na₂HPO₄ (ratio 3:7), 100 mM NaCl, and 1 mM EDTA. The PBS buffer used in broth microdilution and hemolysis assays contained 1.8 mM NaH₂PO₄·H₂O, 10 mM Na₂HPO₄·7H₂O, 135 mM NaCl, and 4.5 mM KCl. The Calcein buffer used in the leakage assay contained 35 mM calcein, 25 mM NaH₂PO₄ and Na₂HPO₄ (ratio 3:7), 20 mM NaCl, and 1 mM EDTA. The pH of all the buffers was adjusted to physiological value 7.4. Mueller-Hinton Broth (MHB) solution was prepared by mixing 5.25 g MHB powder in 250 mL Milli-Q water. Mueller-Hinton Agar (MHA) solution was prepared by mixing 9.50 g MHA powder in 250 mL Milli-Q water.

**Calcein leakage assay.** Pore-forming activity of the peptides was tested by their ability to leak the self-quenching fluorescent dye calcein entrapped within POPC:POPG (1:1 mol/mol) large unilamellar vesicles (LUVs). After mixing DiD to the lipid mixture (in the ratio 1:500 mol/mol), a thin lipid film was created by evaporating of the chloroform, traces of which were removed by desiccating overnight. The lipid film was then dissolved in calcein buffer, followed by vigorous vortexing. The lipid suspension was subsequently subjected to 10 freeze-thaw cycles. To obtain uniform LUVs, the sample was passed 50 times back and forth through an extruder stacked with 100 nm pore size polycarbonate membrane filter. Afterwards, the untrapped calcein was removed on a HiTrap Desalting Column (with Sephadex G-25 Superfine resin) by washing with PBS buffer. The concentration of the lipid suspension was then adjusted to 0.02 mM. The concentration of the peptide solution was 0.1 mM. The inventors monitored the peptide-induced release of calcein from LUVs by the increase in fluorescence intensity after adding increasing amounts of peptides to the LUVs under constant stirring at 25 °C. The investigated range of peptide/lipid molecular ratio was from 1:100 to 1:10. The excitation and emission wavelengths were set to 495 nm and 520 nm, respectively. Maximum fluorescence intensity was obtained by disrupting all the vesicles in the end by adding 10% Triton X-100. Experiments were performed in triplicates with independently prepared lipid vesicles.

**Broth microdilution assay.** Antimicrobial activity of the peptides was investigated against Gram-negative *Escherichia coli* TOP10 strain and Gram-positive *Staphylococcus carnosus* CCM 4838T using broth microdilution assay, based on which the inventors determined the bacterial minimum inhibition concentration (MIC). After cross streaking with bacteria, the petri dishes (with MHA) were incubated for 24 h at 37 °C. Overnight grown cultures were then sub-cultured for 4-6 h at 37 °C in test tubes containing MHB. The sub-cultured solutions were subsequently diluted to a standard concentration of 10⁶ colony forming units (CFU) per mL. Cell counts were determined by measuring the optical density at 600 nm. In a flat-bottomed 96 well plate, the inventors prepared a dilution series for the peptides and for the reference antibiotic ampicillin from 50 to 0.1 µM concentration, which was then inoculated with bacteria and incubated for 24 h at 37 °C. Growth control wells were free from peptides or ampicillin to show the viability of the bacteria, and sterility control wells were free from bacterial inoculation to verify the sterility of the medium. The bacterial growth was visually evaluated based on the turbidity. MIC was determined as the lowest concentrated well where no visible bacterial growth was observed. To test the bacteriostatic or bacteriocidal activity of the peptides, 50 µl solution from the highest concentrated well (i.e., 50 µM) was transferred to a test tube containing 4 mL sterile MHB and incubated for 24 h at 37 °C under constant stirring. Streaking of the same solution was done on MHA containing petri dishes and incubated for 24 h at 37 °C to confirm the morphology of the colonies grown. Experiments were performed in triplicates.

The antimicrobial susceptibility test of the most promising peptide against the recalcitrant ESKAPE pathogens was purchased from Evotec (Macclesfield, UK) for the following strains: *Enterococcus faecium* ATCC 700221, *Staphylococcus aureus* NRS 1, *Klebsiella pneumoniae* ATCC 700603, *Acinetobacter baumanni* GSAB 164, *Pseudomonas aeruginosa* ATCC 13437, *Escherichia coli* ATCC 25922 and *Enterobacter aerogenes* B207A, and was purchased from Essence Line (Praha, Czech Republic) against the following strains: *Enterococcus faecium* ATCC 29212, *Enterococcus hirae* ATCC 10541, *Staphylococcus aureus* ATCC 6538, ATCC 29213, Methicillin-resistant *Staphylococcus aureus* (MRSA) CNCTC 7452, *Klebsiella pneumoniae* ATCC 10031, *Acinetobacter baumanni* ATCC 19606, *Pseudomonas aeruginosa* ATCC 15442 and *Escherichia coli* ATCC 10536 and ATCC 35218. The microdilution assay determining bacterial MIC was similar to the method discussed above with the only exception of the buffer (DMSO 1% (v/v) and 0.1 M PBS) which was used by Evotec to prepare the peptide and ampicillin stock solutions. Moreover, the peptide dilution series were from 16-to-0.03 µM and 50-to-0.8 µM for the experiments conducted by Evotec and Essence Line, respectively. Minimum bactericidal concentration (MBC), i.e., the lowest concentration of the peptide which kills the microorganisms, was determined by sub-culturing the solution from each well on to sterile media at 2, 4, 8, 16, and 32 minutes exposure times, followed by the visual inspection of the bacterial growth (i.e., turbidity in the sub-cultured solution) after incubating for 48 h at 37 °C.

**Hemolysis assay.** Hemolytic toxicity of the peptides was evaluated from their ability to lyse the fresh human red blood cells (HRBCs) collected from the University Hospital (Brno, Czech Republic). To obtain the erythrocyte pellets, the blood samples were centrifuged twice at 700×g and subsequently, once at 1000×g, each for 8 min. at 4 °C, which was followed by thorough washing with PBS. Resuspension of the pellets was done in PBS to obtain 0.5% (vol/vol) RBC suspension. In a well plate with 96 V-shaped bottom wells, the peptides were added in a dilution series from 0.1 to 50 µM concentration. Subsequently, HRBC suspension was added in each well. Negative control wells contained only PBS and HRBC suspension without any peptide (i.e., 0% lysis), and positive control wells included 5 µM melittin in the HRBC suspension. After incubation for 1 h at 37 °C, the well plates were centrifuged at 1000×g for 10 min. at 4 °C. Subsequently, the supernatant was collected in a flat-bottomed well plate and the optical density of the released hemoglobin was measured for each well at 415 nm wavelength. The calculated absorbances from the wells were then normalized against the averaged absorbances of the positive and the negative control wells. EC50 value was calculated from the normalized absorbances indicating 50% hemolysis of HRBCs. Experiments were performed in triplicates with three independent blood samples.

**Neutral red uptake assay.** To evaluate the cytotoxicity of the peptides against human dermal fibroblast (HDF) cell lines, the inventors purchased the neutral red uptake assay from Essence Line (Praha, Czech Republic). The initial cell culture was sub-cultured in supplemented Dulbecco's Modified Eagle Medium (DMEM) and diluted to a cell density of 1 × 10⁵ cells/mL. The diluted culture was then seeded into the wells (1 × 10⁴

## Claims

1. A method for producing an antimicrobial peptide, wherein the peptide comprises a sequence consisting of the following fragments: frag1A-frag2-frag3-frag4-frag5-frag6-frag7-frag8-frag9-frag10;
wherein
the sequences are written from N-terminus to C-terminus,
wherein:
K is lysine;
a is independently selected on each occurence from acidic amino acids selected from D (aspartic acid) and E (glutamic acid);
h is independently selected on each occurence from hydrophobic amino acids selected from A (alanine), M (methionine),V (valine), L (leucine), and I (isoleucine);
p is independently selected on each occurence from polar amino acids selected from T (threonine), Q (glutamine), S (serine), and N (asparagine);
s is independently selected on each occurence from aromatic amino acids W (tryptophan) and F (phenylalanine).
the said method comprising the steps:
- determining the structure of fragment frag1A, wherein frag1A is h;
- determining the structure of fragment frag2, wherein frag2 is selected from KK, aa, ap, pa, and pp;
- determining the structure of fragments frag4 and frag6, wherein
- when frag2 is KK, then frag4 is KK, and frag6 is pph or aph;
- when frag2 is aa, then frag4 is pp or aa;
- when frag4 is pp, then frag6 is Kph;
- when frag4 is aa, then frag6 is pph or Kph;
- when frag2 is ap, then frag4 is pa, and frag6 is Kph;
- when frag 2 is pa, then frag4 is ap, and frag6 is Kph;
- when frag2 is pp, then frag4 is aa, and frag6 is Kph;
- determining the structure of fragment frag3, wherein frag3 is ss or hh.
- determining the structure of fragments frag5 and frag10, wherein
- when frag3 is ss, then frag5 is hhphh (SEQ ID NO. 73), and then frag10 is s;
- when frag3 is hh, then frag5 is hspsh (SEQ ID NO. 74), and then frag10 is h;
- determining the structure of fragment frag7, wherein
- when frag3 is ss, then frag7 is s;
- when frag3 is hh, then
- when frag2 is KK, then frag7 is a or h;
- when frag2 is aa and frag4 is pp, then frag7 is K;
- when frag2 is aa and frag4 is aa, then frag7 is K or h;
- when frag2 is ap, pa, or pp, then frag7 is K;
- determining the structure of fragment frag8, wherein
- when frag2 is KK and frag6 is pph and frag7 is s or a, then frag8 is pph, pah, or aah;
- when frag2 is KK and frag6 is pph and frag7 is h, then frag8 is aah;
- when frag2 is KK and frag6 is aph and frag7 is s or h, then frag8 is pph, pah, or aah;
- when frag2 is KK and frag6 is aph and frag7 is a, then frag8 is pah;
- when frag2 is aa and frag4 is pp, then frag8 is pKh;
- when frag2 is aa and frag4 is aa and frag6 is pph and frag7 is s or h, then frag8 is KKh;
- when frag2 is aa and frag4 is aa and frag6 is pph and frag7 is K, then frag8 is pKh or KKh;
- when frag2 is aa and frag4 is aa and frag6 is Kph, then frag8 is pKh or KKh;
- when frag2 is ap, pa, or pp, then frag8 is pKh;
- determining the structure of fragment frag9, wherein
- when frag2 is KK and frag6 is pph and frag7 is s and frag8 is pph, then frag9 is aa;
- when frag2 is KK and frag6 is pph and frag7 is s and frag8 is pah, then frag9 is ap;
- when frag2 is KK and frag6 is pph and frag7 is s and frag8 is aah, then frag9 is aa;
- when frag2 is KK and frag6 is pph and frag7 is a and frag8 is pph, then frag9 is pa;
- when frag2 is KK and frag6 is pph and frag7 is a and frag8 is pah, then frag9 is aa or pp;
- when frag2 is KK and frag6 is pph and frag7 is a and frag8 is aah, then frag9 is pa;
- when frag2 is KK and frag6 is pph and frag7 is h and frag8 is aah, then frag9 is aa;
- when frag2 is KK and frag6 is aph and frag7 is s and frag8 is pph, then frag9 is pa;
- when frag2 is KK and frag6 is aph and frag7 is s and frag8 is pah, then frag9 is aa or pp;
- when frag2 is KK and frag6 is aph and frag7 is s and frag8 is aah, then frag9 is pa;
- when frag2 is KK and frag6 is aph and frag7 is a and frag8 is pah, then frag9 is pa;
- when frag2 is KK and frag6 is aph and frag7 is h and frag8 is pph, then frag9 is pa;
- when frag2 is KK and frag6 is aph and frag7 is h and frag8 is pah, then frag9 is aa or pp;
- when frag2 is KK and frag6 is aph and frag7 is h and frag8 is aah, then frag9 is pa;
- when frag6 is Kph and frag7 is s and frag8 is pKh, then frag9 is KK;
- when frag6 is Kph and frag7 is K and frag8 is pKh, then frag9 is pK;
- when frag2 is aa and frag4 is aa and frag6 is pph and frag7 is s and frag8 is KKh, then frag9 is KK;
- when frag2 is aa and frag4 is aa and frag6 is pph and frag7 is K and frag8 is pKh, then frag9 is KK;
- when frag2 is aa and frag4 is aa and frag6 is pph and frag7 is K and frag8 is KKh, then frag9 is pK;
- when frag2 is aa and frag4 is aa and frag6 is pph and frag7 is h and frag8 is KKh, then frag9 is KK;
- when frag2 is aa and frag4 is aa and frag6 is Kph and frag7 is s and frag8 is KKh, then frag9 is pK or KK;
- when frag2 is aa and frag4 is aa and frag6 is Kph and frag7 is K and frag8 is KKh, then frag9 is KK;
- when frag2 is aa and frag4 is aa and frag6 is Kph and frag7 is h and frag8 is pKh, then frag9 is KK;
- when frag2 is aa and frag4 is aa and frag6 is Kph and frag7 is h and frag8 is KKh, then frag9 is pK;
- producing the peptide comprising a sequence of fragments frag1A-frag2-frag3-frag4-frag5-frag6-frag7-frag8-frag9-frag10, wherein the fragments are determined in the preceding steps.

2. The method according to claim 1, which is for producing an antimicrobial peptide, wherein the peptide comprises a sequence consisting of fragments: frag1-frag2-frag3-frag4-frag5-frag6-frag7-frag8-frag9-frag10-frag11; wherein
the sequences are written from N-terminal to C-terminal,
wherein:
K is lysine;
a is independently selected on each occurence from acidic amino acids selected from D (aspartic acid) or E (glutamic acid);
h is independently selected on each occurence from hydrophobic amino acids selected from A (alanine) or M (methionine) or V (valine) or L (leucine) or I (isoleucine);
p is independently selected on each occurence from upolar amino acids selected from T (threonine) or Q (glutamine) or S (serine) or N (asparagine);
s is independently selected on each occurence from aromatic amino acids W (tryptophan) or F (phenylalanine).
the said method comprising the steps:
- determining the structure of fragment frag2, wherein frag2 is selected from KK, aa, ap, pa, and pp;
- determining the structure of fragments frag4 and frag6, wherein
- when frag2 is KK, then frag4 is KK, and frag6 is pph or aph;
- when frag2 is aa, then frag4 is pp or aa;
- when frag4 is pp, then frag6 is Kph;
- when frag4 is aa, then frag6 is pph or Kph;
- when frag2 is ap, then frag4 is pa, and frag6 is Kph;
- when frag 2 is pa, then frag4 is ap, and frag6 is Kph;
- when frag2 is pp, then frag4 is aa, and frag6 is Kph;
- determining the structure of fragment frag3, wherein frag3 is ss or hh;
- determining the structure of fragments frag5 and frag10, wherein
- when frag3 is ss, then frag5 is hhphh (SEQ ID NO. 73), and then frag10 is s;
- when frag3 is hh, then frag5 is hspsh (SEQ ID NO. 74), and then frag10 is h;
- determining the structure of fragment frag7, wherein
- when frag3 is ss, then frag7 is s;
- when frag3 is hh, then
- when frag2 is KK, then frag7 is a or h;
- when frag2 is aa and frag4 is pp, then frag7 is K;
- when frag2 is aa and frag4 is aa, then frag7 is K or h;
- when frag2 is ap, pa, or pp, then frag7 is K;
- determining the structure of fragment frag8, wherein
- when frag2 is KK and frag6 is pph and frag7 is s or a, then frag8 is pph, pah, or aah;
- when frag2 is KK and frag6 is pph and frag7 is h, then frag8 is aah;
- when frag2 is KK and frag6 is aph and frag7 is s or h, then frag8 is pph, pah, or aah;
- when frag2 is KK and frag6 is aph and frag7 is a, then frag8 is pah;
- when frag2 is aa and frag4 is pp, then frag8 is pKh;
- when frag2 is aa and frag4 is aa and frag6 is pph and frag7 is s or h, then frag8 is KKh;
- when frag2 is aa and frag4 is aa and frag6 is pph and frag7 is K, then frag8 is pKh or KKh;
- when frag2 is aa and frag4 is aa and frag6 is Kph, then frag8 is pKh or KKh;
- when frag2 is ap, pa, or pp, then frag8 is pKh;
- determining the structure of fragment frag9, wherein
- when frag2 is KK and frag6 is pph and frag7 is s and frag8 is pph, then frag9 is aa;
- when frag2 is KK and frag6 is pph and frag7 is s and frag8 is pah, then frag9 is ap;
- when frag2 is KK and frag6 is pph and frag7 is s and frag8 is aah, then frag9 is aa;
- when frag2 is KK and frag6 is pph and frag7 is a and frag8 is pph, then frag9 is pa;
- when frag2 is KK and frag6 is pph and frag7 is a and frag8 is pah, then frag9 is aa or pp;
- when frag2 is KK and frag6 is pph and frag7 is a and frag8 is aah, then frag9 is pa;
- when frag2 is KK and frag6 is pph and frag7 is h and frag8 is aah, then frag9 is aa;
- when frag2 is KK and frag6 is aph and frag7 is s and frag8 is pph, then frag9 is pa;
- when frag2 is KK and frag6 is aph and frag7 is s and frag8 is pah, then frag9 is aa or pp;
- when frag2 is KK and frag6 is aph and frag7 is s and frag8 is aah, then frag9 is pa;
- when frag2 is KK and frag6 is aph and frag7 is a and frag8 is pah, then frag9 is pa;
- when frag2 is KK and frag6 is aph and frag7 is h and frag8 is pph, then frag9 is pa;
- when frag2 is KK and frag6 is aph and frag7 is h and frag8 is pah, then frag9 is aa or pp;
- when frag2 is KK and frag6 is aph and frag7 is h and frag8 is aah, then frag9 is pa;
- when frag6 is Kph and frag7 is s and frag8 is pKh, then frag9 is KK;
- when frag6 is Kph and frag7 is K and frag8 is pKh, then frag9 is pK;
- when frag2 is aa and frag4 is aa and frag6 is pph and frag7 is s and frag8 is KKh, then frag9 is KK;
- when frag2 is aa and frag4 is aa and frag6 is pph and frag7 is K and frag8 is pKh, then frag9 is KK;
- when frag2 is aa and frag4 is aa and frag6 is pph and frag7 is K and frag8 is KKh, then frag9 is pK;
- when frag2 is aa and frag4 is aa and frag6 is pph and frag7 is h and frag8 is KKh, then frag9 is KK;
- when frag2 is aa and frag4 is aa and frag6 is Kph and frag7 is s and frag8 is KKh, then frag9 is pK or KK;
- when frag2 is aa and frag4 is aa and frag6 is Kph and frag7 is K and frag8 is KKh, then frag9 is KK;
- when frag2 is aa and frag4 is aa and frag6 is Kph and frag7 is h and frag8 is pKh, then frag9 is KK;
- when frag2 is aa and frag4 is aa and frag6 is Kph and frag7 is h and frag8 is KKh, then frag9 is pK;
- determining the structure of fragment frag1, wherein
- when frag2 is KK or pp, then frag1 is KKKKh (SEQ ID NO. 75);
- when frag2 is aa and frag4 is aa, then frag1 is KKKKh (SEQ ID NO. 75);
- when frag2 is aa and frag4 is pp, then frag1 is Kppph (SEQ ID NO. 76);
- when frag2 is ap, then frag1 is KppKh (SEQ ID NO. 77);
- when frag2 is pa, then frag1 is KKpph (SEQ ID NO. 78);
- determining the structure of fragment frag11, wherein
- when frag8 is pph and frag9 is aa, then frag11 is pppK (SEQ ID NO. 79);
- when frag8 is pph and frag9 is pa, then frag11 is KppK (SEQ ID NO. 80);
- when frag8 is pah and frag9 is ap, then frag11 is pKKK (SEQ ID NO. 81);;
- when frag8 is pah or aah and frag9 is aa or pa, then frag11 is KKKK; (SEQ ID NO. 82);
- when frag9 is pp or pK or KK, then frag1 1 is KKKK (SEQ ID NO. 82);
- producing the peptide comprising a sequence of fragments frag1-frag2-frag3-frag4-frag5-frag6-frag7-frag8-frag9-frag10-frag11, wherein the fragments are determined in the preceding steps.

3. The method according to claim 1 or 2, wherein the steps of determining the structure of fragments are computer implemented.

4. A peptide comprising at least 22 amino acids, comprising a sequence selected from the group consisting of:
h KK hh KK hspsh pph a pph pa h (SEQ ID NO. 1)
h aa hh pp hspsh Kph K pKh pK h (SEQ ID NO. 2)
h KK hh KK hspsh pph a pah pp h (SEQ ID NO. 3)
h ap hh pa hspsh Kph K pKh pK h (SEQ ID NO. 4)
h KK hh KK hspsh aph h pph pa h (SEQ ID NO. 5)
h pa hh ap hspsh Kph K pKh pK h (SEQ ID NO. 6)
h KK hh KK hspsh aph h pah pp h (SEQ ID NO. 7)
h pp hh aa hspsh Kph K pKh pK h (SEQ ID NO. 8)
h KK ss KK hhphh pph s pph aa s (SEQ ID NO. 9)
h aa ss pp hhphh Kph s pKh KK s (SEQ ID NO. 10)
h KK ss KK hhphh pph s pah ap s (SEQ ID NO. 11)
h ap ss pa hhphh Kph s pKh KK s (SEQ ID NO. 12)
h KK ss KK hhphh aph s pph pa s (SEQ ID NO. 13)
h pa ss ap hhphh Kph s pKh KK s (SEQ ID NO. 14)
h KK ss KK hhphh aph s pah pp s (SEQ ID NO. 15)
h pp ss aa hhphh Kph s pKh KK s (SEQ ID NO. 16)
h KK hh KK hspsh aph a pah pa h (SEQ ID NO. 17)
h aa hh aa hspsh Kph K pKh pK h (SEQ ID NO. 18)
h KK hh KK hspsh aph h aah pa h (SEQ ID NO. 19)
h aa hh aa hspsh Kph h KKh pK h (SEQ ID NO. 20)
h KK hh KK hspsh aph h pah aa h (SEQ ID NO. 21)
h aa hh aa hspsh Kph h pKh KK h (SEQ ID NO. 22)
h KK hh KK hspsh pph a aah pa h (SEQ ID NO. 23)
h aa hh aa hspsh pph K KKh pK h (SEQ ID NO. 24)
h KK hh KK hspsh pph a pah aa h (SEQ ID NO. 25)
h aa hh aa hspsh pph K pKh KK h (SEQ ID NO. 26)
h KK hh KK hspsh pph h aah aa h (SEQ ID NO. 27)
h aa hh aa hspsh pph h KKh KK h (SEQ ID NO. 28)
h aa hh aa hspsh Kph K KKh KK h (SEQ ID NO. 29)
h KK ss KK hhphh aph s aah pa s (SEQ ID NO. 30)
h aa ss aa hhphh Kph s KKh pK s (SEQ ID NO. 31)
h KK ss KK hhphh aph s pah aa s (SEQ ID NO. 32)
h aa ss aa hhphh Kph s pKh KK s (SEQ ID NO. 33)
h KK ss KK hhphh pph s aah aa s (SEQ ID NO. 34)
h aa ss aa hhphh pph s KKh KK s (SEQ ID NO. 35)
h aa ss aa hhphh Kph s KKh KK s (SEQ ID NO. 36)
wherein
the sequences are written from N-terminus to C-terminus,
wherein
K is lysine;
a is independently selected on each occurence from acidic amino acids selected from D (aspartic acid) and E (glutamic acid);
h is independently selected on each occurence from hydrophobic amino acids selected from A (alanine), M (methionine), V (valine), L (leucine), and I (isoleucine);
p is independently selected on each occurence from polar amino acids selected from T (threonine), Q (glutamine), S (serine) and N (asparagine);
s is independently selected on each occurence from aromatic amino acids W (tryptophan) and F (phenylalanine);
for use in treatment of diseases or infections caused by microbes.

5. The peptide for use according to claim 4, which is extended on N-terminus and/or C-terminus by at least one amino acid, said one or more amino acids are selected from K (lysine) and the amino acids of the groups a (acidic amino acids), b (arginine or histidine), and p (polar amino acids).

6. The peptide for use according to claim 4, which is extended on N-terminus and/or C-terminus by at least four amino acids, wherein the amino acids are selected from K (lysine) and p (polar amino acids).

7. The peptide for use according to claim 4, which has the length of at least 30 amino acids and comprises a sequence selected from the group consisting of:
KKKKh KK hh KK hspsh pph a pph pa h KppK (SEQ ID NO. 37)
Kppph aa hh pp hspsh Kph K pKh pK h KKKK (SEQ ID NO. 38)
KKKKh KK hh KK hspsh pph a pah pp h KKKK (SEQ ID NO. 39)
KppKh ap hh pa hspsh Kph K pKh pK h KKKK (SEQ ID NO. 40)
KKKKh KK hh KK hspsh aph h pph pa h KppK (SEQ ID NO. 41)
KKpph pa hh ap hspsh Kph K pKh pK h KKKK (SEQ ID NO. 42)
KKKKh KK hh KK hspsh aph h pah pp h KKKK (SEQ ID NO. 43)
KKKKh pp hh aa hspsh Kph K pKh pK h KKKK (SEQ ID NO. 44)
KKKKh KK ss KK hhphh pph s pph aa s pppK (SEQ ID NO. 45)
Kppph aa ss pp hhphh Kph s pKh KK s KKKK (SEQ ID NO. 46)
KKKKh KK ss KK hhphh pph s pah ap s pKKK (SEQ ID NO. 47)
KppKh ap ss pa hhphh Kph s pKh KK s KKKK (SEQ ID NO. 48)
KKKKh KK ss KK hhphh aph s pph pa s KppK (SEQ ID NO. 49)
KKpph pa ss ap hhphh Kph s pKh KK s KKKK (SEQ ID NO. 50)
KKKKh KK ss KK hhphh aph s pah pp s KKKK (SEQ ID NO. 51)
KKKKh pp ss aa hhphh Kph s pKh KK s KKKK (SEQ ID NO. 52)
KKKKh KK hh KK hspsh aph a pah pa h KKKK (SEQ ID NO. 53)
KKKKh aa hh aa hspsh Kph K pKh pK h KKKK (SEQ ID NO. 54)
KKKKh KK hh KK hspsh aph h aah pa h KKKK (SEQ ID NO. 55)
KKKKh aa hh aa hspsh Kph h KKh pK h KKKK (SEQ ID NO. 56)
KKKKh KK hh KK hspsh aph h pah aa h KKKK (SEQ ID NO. 57)
KKKKh aa hh aa hspsh Kph h pKh KK h KKKK (SEQ ID NO. 58)
KKKKh KK hh KK hspsh pph a aah pa h KKKK (SEQ ID NO. 59)
KKKKh aa hh aa hspsh pph K KKh pK h KKKK (SEQ ID NO. 60)
KKKKh KK hh KK hspsh pph a pah aa h KKKK (SEQ ID NO. 61)
KKKKh aa hh aa hspsh pph K pKh KK h KKKK (SEQ ID NO. 62)
KKKKh KK hh KK hspsh pph h aah aa h KKKK (SEQ ID NO. 63)
KKKKh aa hh aa hspsh pph h KKh KK h KKKK (SEQ ID NO. 64)
KKKKh aa hh aa hspsh Kph K KKh KK h KKKK (SEQ ID NO. 65)
KKKKh KK ss KK hhphh aph s aah pa s KKKK (SEQ ID NO. 66)
KKKKh aa ss aa hhphh Kph s KKh pK s KKKK (SEQ ID NO. 67)
KKKKh KK ss KK hhphh aph s pah aa s KKKK (SEQ ID NO. 68)
KKKKh aa ss aa hhphh Kph s pKh KK s KKKK (SEQ ID NO. 69)
KKKKh KK ss KK hhphh pph s aah aa s KKKK (SEQ ID NO. 70)
KKKKh aa ss aa hhphh pph s KKh KK s KKKK (SEQ ID NO. 71)
KKKKh aa ss aa hhphh Kph s KKh KK s KKKK (SEQ ID NO. 72)
wherein
the sequences are written from N-terminus to C-terminus,
wherein
K is lysine;
a is independently selected on each occurence from acidic amino acids selected from D (aspartic acid) and E (glutamic acid);
h is independently selected on each occurence from hydrophobic amino acids selected from A (alanine), M (methionine), V (valine), L (leucine), and I (isoleucine);
p is independently selected on each occurence from polar amino acids selected from T (threonine), Q (glutamine), S (serine), and N (asparagine);
s is independently selected on each occurence from aromatic amino acids W (tryptophan) and F (phenylalanine).

8. The peptide for use according to claim 3, which is selected from:
KKKKLKKILKKLFQFINQLDNQLQDIKQNK (SEQ ID NO. 83)
KKKKLKKILKKLWQWINQLDNQLQDIKQNK (SEQ ID NO. 84)
KKKKLKKILKKLFQFINQLENQLQEIKQNK (SEQ ID NO. 85)
KKKKLKKILKKLFQFINQLDNQLQDFKQNK (SEQ ID NO. 86)
KKKKLKKILKKLFQFINQLDNQLQDIKKKK (SEQ ID NO. 87)
KKKKKLKKILKKLFQFINQLDNQLQDIKKKKK (SEQ ID NO. 88) and
KKKKLKKILKKLFQFIDQLDNDLQDIKKKK (SEQ ID NO. 89).

9. Use of the peptide as described in any one of claims 4-8 for killing microbes *in vitro* or *ex vivo.*
